(19) Europäisches Patentamt
European Patent Office
Office européen des brevets

(11) Numéro de publication : **0 107 988**
**B1**

(12) # FASCICULE DE BREVET EUROPÉEN

(45) Date de publication du fascicule du brevet : **29.07.87**

(21) Numéro de dépôt : **83401914.3**

(22) Date de dépôt : **29.09.83**

(51) Int. Cl.[4] : **C 07 D471/04**, A 61 K 31/47 // (C07D471/04, 235:00, 221:00)

(54) **Nouvelles imidazo/1,2-a/ quinoléines et leurs sels, leur préparation, leur application à titre de médicaments et les compositions les renfermant.**

(30) Priorité : **01.10.82 GB 8228069**

(43) Date de publication de la demande : **09.05.84 Bulletin 84/19**

(45) Mention de la délivrance du brevet : **29.07.87 Bulletin 87/31**

(84) Etats contractants désignés : **AT BE CH DE FR IT LI LU NL SE**

(56) Documents cités :
**EP-A- 0 062 580**
**FR-A- 2 378 031**

(73) Titulaire : **ROUSSEL-UCLAF**
**35, boulevard des Invalides**
**F-75007 Paris (FR)**

(72) Inventeur : **Tully, Wilfred Roger**
**5 Saint Peter's Road**
**Cirencester Gloucestershire (GB)**

(74) Mandataire : **Vieillefosse, Jean-Claude et al**
**Département des Brevets ROUSSEL UCLAF B.P no 9**
**F-93230 Romainville (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

EP 0 107 988 B1

**Description**

La présente invention concerne de nouvelles imidazo [1,2-a] quinoléines et leurs sels, leur préparation, leur application à titre de médicaments, les compositions les renfermant et les nouveaux intermédiaires obtenus.

L'invention a pour objet de nouvelles imidazo [1,2-a] quinoléines et leurs sels, caractérisées en ce qu'elles répondent à la formule générale (I) :

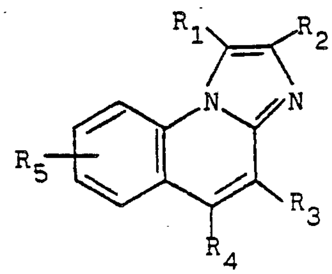

(I)

dans laquelle $R_1$ représente un atome d'hydrogène ou un atome de chlore ou de brome, $R_2$ représente un radical benzoyle, un radical

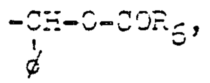

dans lequel $R_6$ représente un radical alcoyle renfermant de 1 à 5 atomes de carbone, un radical :

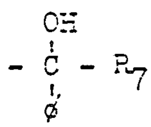

dans lequel $R_7$ représente un radical alcoyle renfermant de 1 à 5 atomes de carbone, ou un radical :

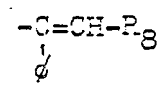

dans lequel $R_8$ représente un atome d'hydrogène ou un radical alcoyle renfermant de 1 à 4 atomes de carbone, le symbole $\phi$ représentant un radical phényle, $R_3$ représente un atome d'hydrogène ou un radical alcoyle renfermant de 1 à 5 atomes de carbone,

$R_4$ représente un radical alcoxy renfermant de 1 à 8 atomes de carbone, un radical hydroxy, un radical acyloxy renfermant de 2 à 8 atomes de carbone, un radical alcoyle renfermant de 1 à 8 atomes de carbone,

$R_5$ représente un radical alcoyle renfermant de 1 à 8 atomes de carbone, étant entendu que $R_1$ et $R_3$ ne peuvent représenter un atome d'hydrogène lorsque $R_2$ représente un radical benzoyle et $R_4$ représente un radical alcoxy renfermant de 1 à 8 atomes de carbone, ces produits étant décrits dans la demande EP-A-0 062 580 au nom de la demanderesse.

Dans la formule générale (I) et dans ce qui suit, le terme radical alcoyle renfermant de 1 à 5 atomes de carbone désigne, par exemple, un radical méthyle, éthyle, propyle, isopropyle, butyle, isobutyle ou pentyle ; le terme radical alcoxy renfermant de 1 à 8 atomes de carbone désigne, par exemple, un radical méthoxy, éthoxy, propoxy, isopropoxy, butoxy, isobutoxy ou octyloxy ; le terme radical acyloxy renfermant de 2 à 8 atomes de carbone désigne, par exemple, un radical acétoxy propionyloxy, butyryloxy ; le terme radical alcoyle renfermant de 1 à 8 atomes de carbone désigne par exemple, un radical méthyle, éthyle, propyle, isopropyle, butyle, pentyle, ou octyle ;

Lorsque $R_2$ est différent d'un radical benzoyle, il peut représenter par exemple, un radical acétoxy 1-phényl méthyle, 1-phényl vinyle ou 1-hydroxy 1-phényl éthyle.

Les sels d'addition avec les acides minéraux ou organiques peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, alcanesulfoniques, tels que l'acide méthane sulfonique et arylsulfoniques, tels que l'acide benzène sulfonique.

Parmi les produits, objets de l'invention, on peut citer notamment les dérivés répondant à la formule

(I) ci-dessus, ainsi que leurs sels, caractérisés en ce que dans ladite formule (I), $R_3$ représente un atome d'hydrogène ou un radical méthyle ou éthyle, $R_4$ représente un radical méthoxy, hydroxy, acétoxy ou méthyle, $R_5$ représente un radical méthyle ou éthyle, $R_1$ et $R_2$ ayant la signification déjà indiquée.

Parmi les produits, objets de l'invention, on retient plus particulièrement les dérivés répondant à la formule (I) ci-dessus ainsi que leurs sels, caractérisés en ce que dans ladite formule (I), $R_1$ représente un atome d'hydrogène, $R_2$ représente un radical benzoyle, un radical 1-phényl vinyle, $R_3$ représente un atome d'hydrogène, un radical méthyle ou éthyle, $R_4$ représente un radical méthoxy, hydroxy, acétoxy ou méthyle, $R_5$ représente un radical éthyle.

Parmi ces derniers, on retient tout particulièrement les produits de formule (I) dans laquelle $R_1$ représente un atome d'hydrogène, $R_2$ représente un radical benzoyle, $R_3$ représente un radical méthyle ou éthyle, $R_4$ représente un radical méthoxy $R_5$ représente un radical éthyle ainsi que leurs sels et notamment la (7-éthyl 5-méthoxy 4-méthyl [imidazo] 1,2-a [quinoléin-2-yl]) phényl méthanone et la (4,7-diéthyl 5-méthoxy [imidazo] 1,2-a [quinoléin-2-yl]) phényl méthanone ainsi que leurs sels.

L'invention a également pour objet, un procédé de préparation des produits de formule (I) dans laquelle $R_1$ représente un atome d'hydrogène, $R_2$ représente un radical benzoyle, $R_4$ représente un radical $R_4$ lequel représente un radical alcoxy renfermant de 1 à 8 atomes de carbone ou un radical alcoyle renfermant de 1 à 8 atomes de carbone, $R_3$ et $R_5$ ont la signification déjà indiquée, ainsi que de leurs sels, caractérisé en ce que l'on fait réagir un produit de formule (II) :

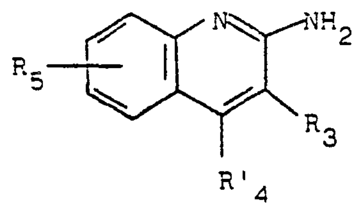

(II)

dans laquelle $R_3$, $R'_4$ et $R_5$ ont la signification déjà indiquée, avec un produit de formule (III) :

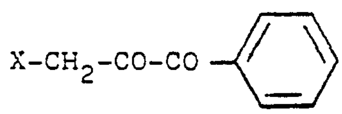

(III)

dans laquelle X représente un atome d'halogène, pour obtenir un produit de formule (IV) :

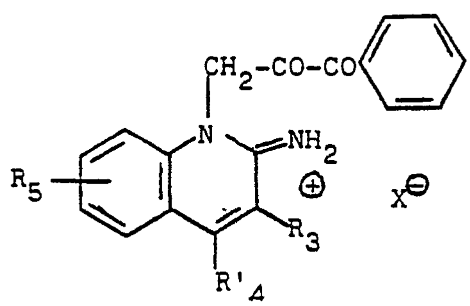

(IV)

dans laquelle $R_3$, $R'_4$ et $R_5$ ont la signification déjà indiquée, puis cyclise le produit de formule (IV) obtenu pour former le produit de formule ($I_A$) :

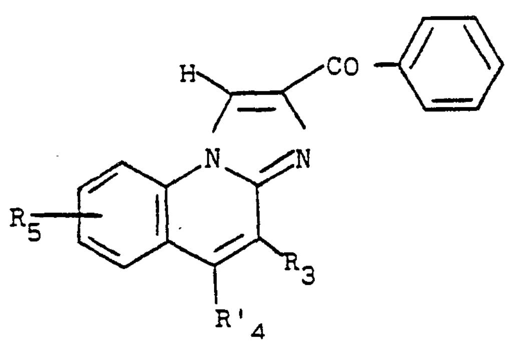

($I_A$)

dans laquelle $R_3$, $R'_4$ et $R_5$ ont la signification précitée que l'on salifie le cas échéant.

Dans les conditions préférentielles de mise en œuvre de l'invention, le procédé de préparation ci-dessus décrit est caractérisé en ce que :

a) la réaction du produit de formule (II) avec le produit de formule (III) est effectuée au sein d'un solvant organique tel que le diméthoxyméthane ;

b) la cyclisation du produit de formule (IV) est effectuée par chauffage au sein d'un solvant organique tel que l'éthanol ;

c) la réaction du produit de formule (II) avec le produit de formule (III) est poursuivie jusqu'au produit de formule ($I_A$) sans isoler le produit de formule (IV).

On peut avantageusement préparer les sels d'addition des produits de formule (I) en faisant réagir, en proportions sensiblement stœchiométriques, un acide minéral ou organique avec lesdits produits de formule (I). Les sels peuvent être préparés sans isoler les bases correspondantes.

L'invention a également pour objet un procédé de préparation des produits de formule (I) dans laquelle $R_1$ représente un atome de chlore ou de brome, $R_2$ représente un radical benzoyle ainsi que de leurs sels, caractérisé en ce que l'on halogène un produit correspondant dans lequel $R_1$ représente un atome d'hydrogène, pour obtenir un produit de formule ($I_B$) :

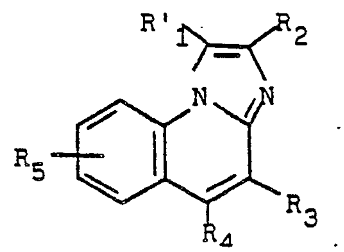

($I_B$)

dans laquelle $R'_1$ représente un atome de chlore ou de brome, $R_2$, $R_3$, $R_4$ et $R_5$ ayant la signification déjà indiquée, que l'on isole, et, si désiré, salifie.

Dans les conditions préférentielles de mise en œuvre de l'invention, le procédé de préparation ci-dessus décrit est caractérisé en ce que :

a) l'halogénation est effectuée au moyen du N-bromosuccinimide lorsque l'on désire préparer un produit de formule (I) dans laquelle $R_1$ représente un atome de brome,

b) l'halogénation est effectuée au moyen du N-chlorosuccinimide lorsque l'on désire préparer un produit de formule (I) dans laquelle $R_1$ représente un atome de chlore.

L'invention a encore pour objet un procédé de préparation des produits de formule (I) dans laquelle $R_4$ représente un radical $R'_4$, $R_1$, $R_3$, $R'_4$ et $R_5$ ont la signification déjà indiquée et $R_2$ représente un radical benzoyle ainsi que de leurs sels, caractérisé en ce que l'on réduit un produit de formule (V) :

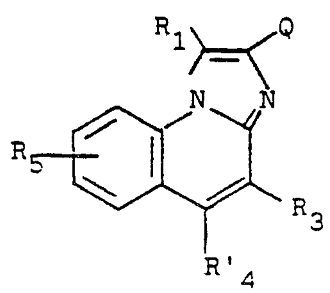

(V)

dans laquelle $R_1$, $R_3$, $R'_4$ et $R_5$ ont la signification déjà indiquée et Q représente un groupement alcoxycarbonyl renfermant de 2 à 4 atomes de carbone ou aralcoxycarbonyl renfermant de 8 à 12 atomes de carbone, pour obtenir un produit de formule (VI) :

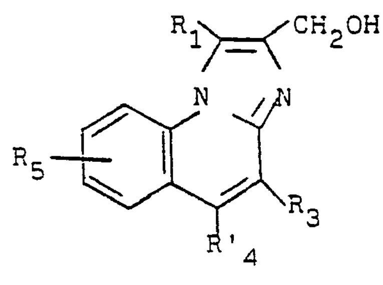

(VI)

dans laquelle $R_1$, $R_3$, $R'_4$ et $R_5$ ont la signification déjà indiquée, que l'on oxyde pour obtenir un produit de formule (VII) :

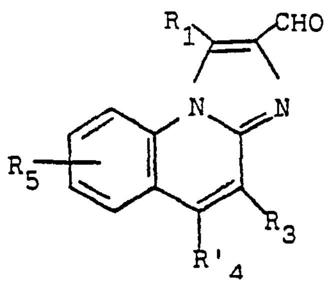

(VII)

dans laquelle $R_1$, $R_3$, $R'_4$ et $R_5$ ont la signification déjà indiquée, que l'on soumet à l'action d'un réactif de Grignard formé à partir d'un produit de formule (VIII) :

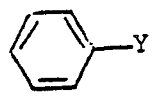

(VIII)

dans laquelle Y représente un atome de chlore, d'iode ou de brome ou à l'action du phényllithium, pour obtenir un produit de formule (IX) :

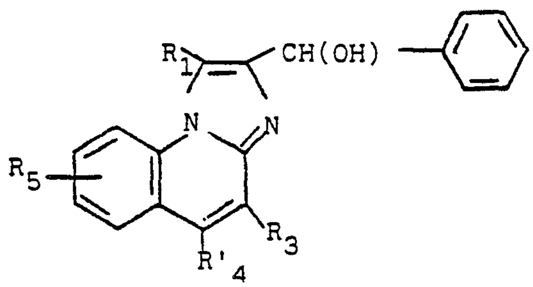

(IX)

dans laquelle $R_1$, $R_3$, $R'_4$ et $R_5$ ont la signification déjà indiquée, que l'on oxyde pour obtenir un produit de formule ($I_C$) :

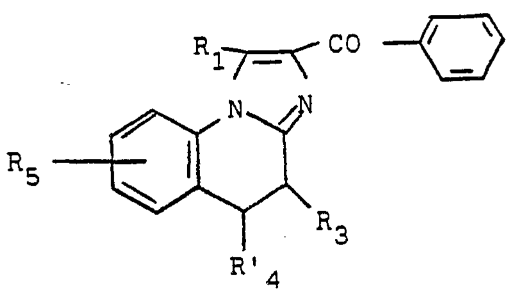

($I_C$)

dans laquelle $R_1$, $R_3$, $R'_4$ et $R_5$ ont la signification déjà indiquée, que l'on isole et, si désiré, salifie.

Dans les conditions préférentielles de mise en œuvre de l'invention, le procédé de préparation ci-dessus décrit est caractérisé en ce que :

a) la réduction du produit de formule (V) est effectuée par l'hydrure de lithium-aluminium, le mélange borohydrure de sodium-chlorure d'aluminium ou le borohydrure de lithium ;

b) l'oxydation du produit de formule (VI) est effectuée à l'aide de dioxyde de manganèse ou encore d'acide nitrique, de chlorure ferrique, de mélange oxyde de chrome-pyridine ou encore par la méthode d'Oppenauer ou par déshydrogénation sur un catalyseur à base de cuivre,

c) la réaction du produit de formule (VII) avec le réactif de Grignard ou avec le phényllithium est effectuée au sein du tétrahydrofuranne,

d) l'oxydation du produit de formule (IX) est effectuée par l'une des méthodes indiquées ci-dessus.

Les produits de départ de formule (V) peuvent en général être préparés selon le procédé décrit dans le brevet anglais 1 596 652 ou par des procédés analogues.

L'invention a encore pour objet un procédé de préparation des produits de formule (I) dans laquelle $R_2$ représente un radical :

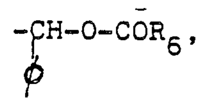

$R_1$, $R_3$, $R_5$ étant définis comme précédemment et $R_4$ étant différent d'hydroxy ainsi que de leurs sels, caractérisé en ce que l'on réduit un produit de formule :

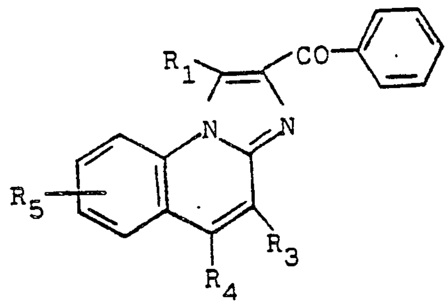

(A)

dans laquelle $R_1$, $R_3$, $R_4$ et $R_5$ sont définis comme précédemment, pour obtenir un produit de formule (X) :

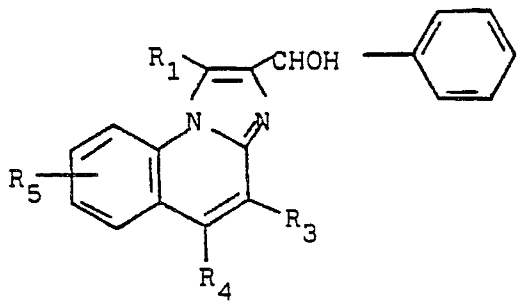

(X)

dans laquelle $R_1$, $R_3$, $R_4$ et $R_5$ sont définis comme précédemment, que l'on soumet à une réaction d'acylation, pour obtenir un produit de formule ($I_D$) :

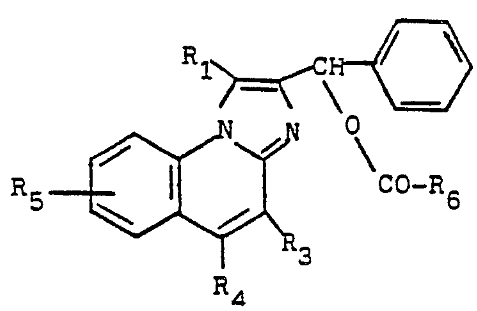

($I_D$)

dans laquelle $R_1$, $R_3$, $R_4$ et $R_5$ ont la signification déjà indiquée que l'on isole et, si désiré, salifie.

Dans les conditions préférentielles de mise en œuvre de l'invention, le procédé de préparation ci-dessus décrit est caractérisé en ce que :

a) la réduction du produit de formule (A) est effectuée au moyen du borohydrure de sodium au sein d'un solvant organique tel que l'éthanol,

b) l'acylation du produit de formule (X) est effectuée au moyen d'un halogénure d'acyle, notamment un chlorure ou un bromure au sein de la pyridine.

La formule (A) englobe à la fois les produits de formule ($I_A$), certains produits de formule ($I_B$) et certains produits décrits dans la demande EP-A-0 062 580 déposée par la demanderesse dont la préparation est donnée plus loin.

L'invention a encore pour objet un procédé de préparation des produits de formule (I) dans laquelle $R_2$ représente un radical

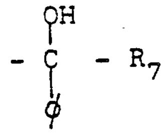

ou un radical

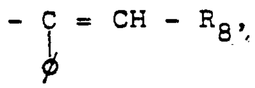

$R_1$, $R_3$, $R_5$, $R_7$ et $R_8$ étant définis comme précédemment et $R_4$ est différent d'acyloxy, caractérisé en ce que l'on fait réagir un produit de formule (A) tel que défini précédemment, avec un réactif de Grignard formé à partir d'un produit de formule (XI) :

$$Y—R_7 \quad \text{(XI)}$$

ou avec un réactif de formule $R_7$—Li dans lesquelles Y et $R_7$ sont définis comme précédemment, pour obtenir un produit de formule $I_E$ :

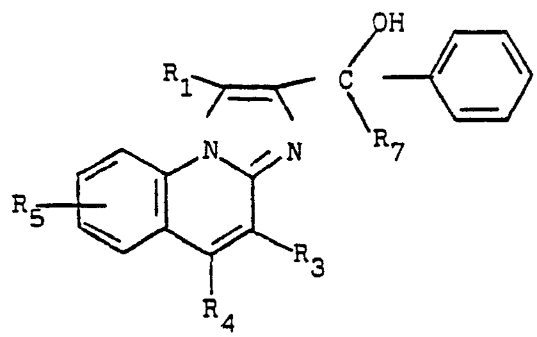

($I_E$)

dans laquelle $R_1$, $R_3$, $R_4$, $R_5$ et $R_7$ ont la signification déjà indiquée et que,

soit on isole et si désiré, salifie le produit de formule (I) ainsi obtenu,

soit on déshydrate ce dernier, dans le cas où $R_7$ représente un radical $—CH_2R_8$, $R_8$ étant défini comme précédemment, pour obtenir un produit de formule ($I_F$) :

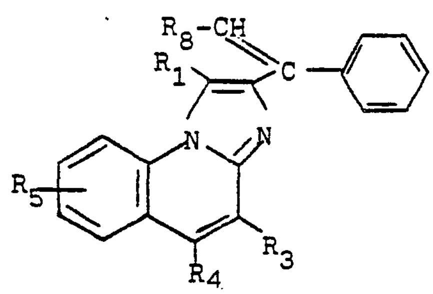

($I_F$)

dans laquelle $R_1$, $R_3$, $R_4$, $R_5$ et $R_8$ ont la signification déjà indiquée, que l'on isole et, si désiré, salifie.

Dans les conditions préférentielles de mise en œuvre de l'invention, le procédé de préparation ci-dessus décrit est caractérisé en ce que :

a) la réaction du produit de formule (A) avec le produit de formule XI ou avec le réactif de formule $R_7$-Li est effectuée au sein de l'éther anhydre,

b) la déshydratation du produit de formule ($I_D$) est effectuée au moyen d'acide chlorhydrique concentré.

L'invention a encore pour objet un procédé de préparation des produits de formule (I) dans laquelle

$R_1$, $R_3$ et $R_5$ sont définis comme précédemment, $R_2$ représente un radical benzoyle ou un groupement

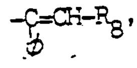

$R_8$ étant défini comme précédemment et $R_4$ représente un radical hydroxy ou un radical acyloxy renfermant de 2 à 8 atomes de carbone, ainsi que de leurs sels, caractérisé en ce que l'on hydrolyse un produit correspondant dans lequel $R_4$ représente un radical alcoxy renfermant de 1 à 8 atomes de carbone, pour obtenir un produit de formule ($I_G$) :

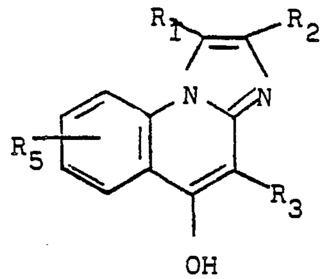

($I_G$)

dans laquelle $R_1$, $R_2$, $R_3$ et $R_5$ ont la signification déjà indiquée et que,

soit on isole et, si désiré, salifie le produit de formule ($I_G$) ainsi obtenu,

soit on fait réagir ce dernier avec un halogénure de formule (XII) :

$$Hal—R_9 \quad (XII)$$

dans laquelle $R_9$ représente un radical acyle renfermant de 2 à 8 atomes de carbone et Hal représente un atome de chlore ou de brome, ou avec un anhydride d'acide de formule (XIII) :

$$(R_9)_2O \quad (XIII)$$

dans laquelle $R_9$ a la signification déjà indiquée, pour obtenir un produit de formule ($I_H$) :

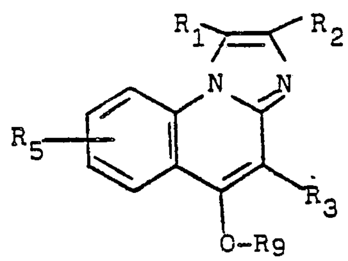

($I_H$)

dans laquelle $R_1$, $R_2$, $R_3$, $R_9$ et $R_5$ ont la signification déjà indiquée que l'on isole et, si désiré, salifie.

Dans les conditions préférentielles de mise en œuvre de l'invention, le procédé de préparation ci-dessus décrit est caractérisé en ce que :

a) l'hydrolyse du produit dans lequel $R_4$ représente un radical alcoxy est effectuée au moyen de l'acide bromhydrique par chauffage au reflux du milieu réactionnel pendant trente heures en présence d'eau ;

b) la réaction du produit de formule ($I_G$) avec l'halogénure de formule (XII) ou l'anhydride d'acide de formule (XIII) est effectuée au sein de la triéthylamine.

Les produits obtenus par le procédé, objet de la présente invention, possèdent de très intéressantes propriétés pharmacologiques ; ils sont doués notamment de remarquables propriétés anxiolytiques et d'une certaine activité hypnotique.

Ces propriétés sont illustrées plus loin dans la partie expérimentale.

Ces propriétés justifient l'utilisation des nouvelles imidazo [1,2-a] quinoléines de formule (I) ainsi que de leurs sels pharmaceutiquement acceptables, à titre de médicaments.

La présente invention a ainsi également pour objet l'application à titre de médicaments des nouvelles imidazo [1,2-a] quinoléines telles que définies par la formule générale (I) ainsi que de leurs sels d'addition

avec les acides pharmaceutiquement acceptables.

Parmi les médicaments, objet de l'invention, on retient notamment, les médicaments caractérisés en ce qu'ils sont constitués par les nouvelles imidazo [1,2-a] quinoléines répondant à la formule (I) dans laquelle $R_3$ représente un atome d'hydrogène ou un radical méthyle ou éthyle, $R_4$ représente un radical méthoxy, hydroxy, acétoxy ou méthyle, $R_5$ représente un radical méthyle ou éthyle, $R_1$ et $R_2$ ayant la signification déjà indiquée ainsi que par leurs sels d'addition avec les acides pharmaceutiquement acceptables.

Parmi les médicaments, objet de l'invention, on retient plus particulièrement, les médicaments caractérisés en ce qu'ils sont constitués par les nouvelles imidazo [1,2-a] quinoléines répondant à la formule (I) dans laquelle $R_1$ représente un atome d'hydrogène, $R_2$ représente un radical benzoyle, un radical 1-phényl vinyle, $R_3$ représente un atome d'hydrogène un radical méthyle ou éthyle, $R_4$ représente un radical méthoxy, hydroxy, acétoxy ou méthyle, $R_5$ représente un radical éthyle ainsi que par leurs sels d'addition avec les acides pharmaceutiquement acceptables.

Parmi les médicaments préférés de l'invention, on retient tout particulièrement ceux répondant à la formule (I) dans laquelle $R_1$ représente un atome d'hydrogène, $R_2$ représente un radical benzoyle, $R_3$ représente un radical méthyle ou éthyle, $R_4$ représente un radical méthoxy, $R_5$ représente un radical éthyle, ainsi que leurs sels d'addition avec les acides pharmaceutiquement acceptables, et notamment :

la (7-éthyl 5-méthoxy 4-méthyl [imidazo] 1,2a [quinoléin-2-yl]) phényl méthanone,

la (4,7-diéthyl 5-méthoxy [imidazo] 1,2-a [quinoléin-2-yl]) phényl méthanone.

Ces médicaments trouvent, par exemple, leur emploi dans le traitement des états anxieux, des anxiétés chroniques avec agitation, irritabilité, agressivité, des anxiétés avec insomnies et tensions musculaires, des angoisses.

La dose usuelle, variable selon le produit utilisé, le sujet traité et l'affection en cause peut être, par exemple, de 0,1 mg à 200 mg par jour, par voie orale.

L'invention a également pour objet les compositions pharmaceutiques qui renfermant au moins un dérivé précité ou l'un de ses sels d'addition avec les acides pharmaceutiquement acceptables à titre de principe actif.

A titre de médicaments, les dérivés répondant à la formule (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme, par exemple, les comprimés, simples ou dragéifiés, les gélules, les capsules, les granulés, les suppositoires, les préparations injectables, elles sont préparées selon les méthodes usuelles. Le ou les principes actifs peuvent être incorporés à des excipients habituellement employés dans ces compositions pharmaceutiques tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les produits intermédiaires de formule (X) :

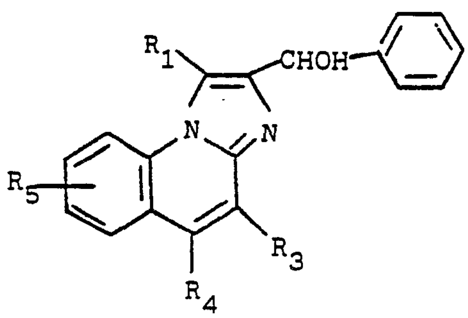

(X)

dans laquelle $R_1$, $R_3$, $R_4$ et $R_5$ ont la signification déjà indiquée sont des produits nouveaux.

Les produits de formule (II) lorsqu'ils ne sont pas connus, peuvent être préparés par un procédé analogue à celui décrit dans le brevet GB-1596652 ou dans le brevet GB-1542778. Les produits de formule (II) peuvent également être préparés comme indiqué dans J.C.S. 1958 page 614 et suivantes ou dans Synthesis 1977 p. 500.

Les produits de formule (A) décrits dans la demande EP-A-0062580 précitée, peuvent être préparés par un procédé qui consiste principalement à faire réagir un produit de formule :

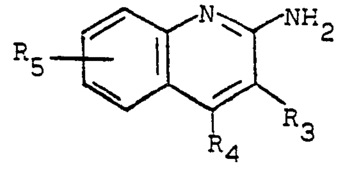

dans laquelle $R_3$, $R_4$ et $R_5$ ont la signification déjà indiquée, avec un produit de formule :

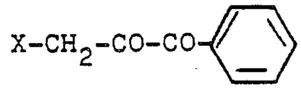

dans laquelle X représente un atome d'halogène, pour obtenir un produit de formule :

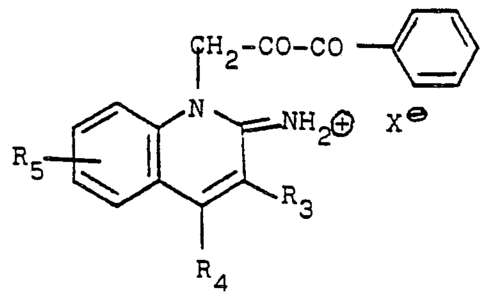

dans laquelle $R_3$, $R_4$, $R_5$ et X ont la signification déjà indiquée, puis à cycliser le produit obtenu, pour former le produit de formule générale (A) recherché.

Il va être donné maintenant à titre non limitatif des exemples de mise en œuvre de l'invention.

Exemple 1 : Acétate de (7-éthyl 5-méthoxyimidazo [1,2-a] quinoléin-2-yl) phénylméthyle

Stade A

(7-éthyl 5-méthoxyimidazo [1,2-a] quinoléin-2-yl) phényl méthanol

On ajoute par petites portions 1,51 g de borohydrure de sodium à une suspension maintenue sous agitation, de 3,3 g de (7-méthyl 5-méthoxyimidazo [1,2-a] quinoléin-2-yl) phénylméthanone dans 200 $cm^3$ d'éthanol. On maintient sous agitation à température ambiante pendant deux heures, détruit l'excès de borohydrure de sodium par addition d'eau puis évapore le solvant sous pression réduite.

On ajoute au résidu un mélange eau-chlorure de méthylène, décante, extrait de phase aqueuse au chlorure de méthylène, réunit les phases organiques, les lave à l'eau, les sèche et évapore le solvant.

On obtient 3,10 g de produit attendu.

Stade B

Acétate de (7-éthyl 5-méthoxyimidazo [1,2-a] quinoléin-2-yl) phénylméthyl

On met en solution 3,1 g de produit obtenu au stade A dans 50 $cm^3$ de pyridine, refroidit à 5 °C et ajoute lentement 1,10 g de chlorure d'acétyle, tout en maintenant la température à 5 °C maximum.

Après l'addition du chlorure d'acétyle, on laisse la température remonter à la température ambiante, puis verse le mélange dans un mélange eau-glace.

On filtre les cristaux de produit obtenu, les lave à l'eau les sèche et les recristallise dans un mélange éther de pétrole-acétate d'éthyle.

On obtient 2,23 g de produit attendu. F = 93-95 °C.

Exemple 2 : (1-bromo 7-éthyl 5-méthoxyimidazo [1,2-a] quinoléin-2-yl) phénylméthanone

On ajoute 1 g de N-bromosuccimide à une solution maintenue sous agitation de 1,6 g de (7-éthyl 5-méthoxyimidazo [1,2-a] quinoléin-2-yl) phénylméthanone dans 25 $cm^3$ de chloroforme.

Dix minutes plus tard, on lave la solution à l'eau, l'évapore à sec et triture le résidu dans l'éthanol.

On obtient 1,4 g de produit attendu. F = 171-172 °C.

Exemple 3 : (1-chloro 7-éthyl 5-méthoxyimidazo [1,2-a] quinoléin-2-yl) phénylméthanone

En utilisant une méthode analogue à celle décrite à l'exemple 2, mais en utilisant le N-chlorosuccinimide (0,8 g) et en portant la solution au reflux pendant quatre heures, on obtient 1,4 g de produit attendu. F = 176-178 °C.

Exemple 4 : 2-benzoyl 7-éthylimidazo [1,2-a] quinoléin-5-ol

On met en suspension 10 g de (7-éthyl 5-méthoxyimidazo [1,2-a] quinoléin-2-yl) phénylméthanone dans 200 $cm^3$ d'acide bromhydrique à 48 %.

On porte au reflux pendant 30 heures, dilue à l'eau, refroidit à 0 °C et filtre. On lave les cristaux obtenus à l'eau puis les dissout à chaud dans le méthanol, on ajoute à la solution de l'ammoniaque, puis dilue à l'eau et essore les cristaux formés.

On obtient 7,5 g de produit attendu. F = 268-270 °C.

Exemple 5 : 1-(7-éthyl 5-méthoxyimidazo [1,2-a] quinoléin-2-yl) 1-phényléthanol

On ajoute par petites portions, 6,6 g de (7-éthyl 5-méthoxy imidazo [1,2-a] quinoléin-2-yl) phénylméthanone à une solution maintenue sous agitation d'iodure de méthyle-magnésium (préparée à partir de 1,2 g de magnésium) et de iodométhane (7,1 g) dans 200 $cm^3$ d'éther éthylique anhydre.

On porte le mélange au reflux pendant huit heures et verse dans une solution aqueuse de chlorure d'ammonium.

On extrait à l'acétate d'éthyle, décante, lave la phase organique à l'eau, la sèche et l'évapore à sec. On cristallise le résidu dans l'éther et obtient 4,9 g de produit attendu. F = 187-188 °C.

Exemple 6 : Acétate de 2-benzoyl 7-éthylimidazo [1,2-a] quinoléin-2-yle

On chauffe au bain de vapeur pendant un heure un mélange de 2 g de produit obtenu à l'exemple 4, 10 $cm^3$ d'anhydride acétique et 5 gouttes de triéthylamine. On maintient ensuite sous agitation à température ambiante pendant une nuit, dilue à l'éther et filtre les cristaux formés.

On obtient 2 g de produit attendu. F = 202-203 °C.

Exemple 7 : Chlorhydrate de 7-éthyl 5-méthoxy 2-(1-phénylvinyl) imidazo [1,2-a] quinoléine

On ajoute par petites portions 2,5 g de produit obtenu à l'exemple 5 dans 15 $cm^3$ d'acide chlorhydrique concentré auquel on avait ajouté 1 $cm^3$ d'éthanol et que l'on a porté à 80 °C.

Après une heure, on dilue le mélange à l'eau, filtre, lave les cristaux à l'eau et à l'acétone et obtient 2,6 g de produit attendu. F = 188-191 °C.

Exemple 8 : (7-éthyl 5-méthoxy 4-méthylimidazo [1,2-a] quinoléin-2-yl) phénylméthanone

On dissout de la 2-amino 6-éthyl 4-méthoxy 3-méthylquinoléine dans le chlorure de méthylène, puis ajoute de la 3-bromophényl propan-1,2-dione et maintient le mélange sous agitation à température ambiante pendant deux heures et demie. On filtre le sel cristallisé constitué par du bromure de 6-éthyl 4-méthoxy 3-méthyl 1-(3-phényl 2,3-dioxopropyl) 2-quinoliniminium, le lave à l'éther, le met en suspension dans l'éthanol et le porte au reflux jusqu'à obtention d'une solution claire. On refroidit à température ambiante, filtre les cristaux, concentre les liqueurs-mères sous pression réduite et les dilue à l'éther. On obtient un second jet de produit que l'on combine avec le premier et que l'on mélange à du chloroforme et à une solution aqueuse de carbonate de potassium.

On décante, lave la phase organique, la sèche, la concentre sous pression réduite, la dilue à l'éthanol. On obtient ainsi avec un rendement de 22 % le produit attendu. F = 141-142 °C.

Exemple 9 : (7-éthyl 5-méthylimidazo [1,2-a] quinoléin-2-yl) phényl méthanol

On utilise une méthode analogue à celle décrite à l'exemple 8 mais en utilisant au départ la 2-amino 6-éthyl 4-méthylquinoléine et la 3-bromo 1-phénylpropane-1,2-dione.

On obtient le produit attendu avec un rendement de 22 %. F = 187-189 °C.

Exemple 10 : (4,7-diéthyl 5-méthoxyimidazo [1,2-a] quinoléin-2-yl) phénylméthanone

En utilisant une méthode analogue à celle décrite à l'exemple 8, mais en utilisant au départ la 2-amino 3,6-diéthyl 4-méthoxyquinoléine et la 3-bromo 1-phénylpropane-1,2-dione, on obtient le produit attendu avec un rendement de 18 %. F = 145-146 °C.

Dans le tableau I ci-après sont fournis notamment les points de fusion des produits des exemples 1 à 10, ainsi que les spectres I.R. et les résultats des microanalyses qui ont été effectués sur ces produits.

(Voir Tableau I page 12)

Etude pharmacologique

L'affinité des composés pour les récepteurs des benzodiazépines a été évaluée en utilisant un radioligand [$^3$H] flunitrazépam et la méthode de Squires et Braestrup (Nature, 1977, 266, 732) modifiée.

Tableau 1

| Exemple | $R_1$ | $R_2$ | $R_3$ | $R_4$ | $R_5$ | Rend. | F°C | IR (KBr) | Formule | Poids mol. | Calculé/Trouvé C | H | N | X |
|---|---|---|---|---|---|---|---|---|---|---|---|---|---|---|
| 1 | H | CH(OAc)Ph | H | $OCH_3$ | 7-$C_2H_5$ | 60 | 93-5 | 3140,1630 | $C_{23}H_{22}N_2O_3$ | 374,45 | 73,78<br>73,9 | 5,92<br>6,0 | 7,48<br>7,5 | |
| 2 | Br | CO Ph | H | $OCH_3$ | " | 71 | 171-2 | 1662,1638 | $C_{21}H_{17}BrN_2O_2$ | 409,29 | 61,63<br>61,7 | 4,19<br>4,2 | 6,81<br>6,9 | 19,52(Br)<br>19,6 |
| 3 | Cl | CO Ph | H | $OCH_3$ | " | 79 | 176-8 | 1640 | $C_{21}H_{17}ClN_2O_2$ | 364,83 | 69,14<br>69,0 | 4,70<br>4,8 | 7,68<br>7,7 | 9,72(Cl)<br>10,1 |
| 4 | H | CO Ph | H | OH | " | 71 | 268-70 | 3130,1638 | $C_{20}H_{16}N_2O_2$ | 316,36 | 75,93<br>75,65 | 5,10<br>5,15 | 8,85<br>8,9 | |
| 5 | H | C(OH)MePh | H | $OCH_3$ | " | 78 | 187-8 | 1741,1630 | $C_{22}H_{22}N_2O_2$ | 346,44 | 76,28<br>76,0 | 6,40<br>6,5 | 8,09<br>8,0 | |
| 6 | H | CO Ph | H | OAc | " | 71 | 202-3 | 3115,1771<br>1645 | $C_{22}H_{18}N_2O_3$ | 358,40 | 73,73<br>73,7 | 5,06<br>5,1 | 7,82<br>7,8 | |
| 7 | H | CPh=$CH_2$ | H | $OCH_3$ | " | 99 | 188-91 | 1641,<br>1630infl.<br>1620infl. | $C_{22}H_{20}N_2OHCl$<br>(1½ $H_2O$) | 364,9 | 67,43<br>67,4 | 5,91<br>6,2 | 7,15<br>7,2 | 9,05(Cl)<br>9,2 |
| 8 | H | CO Ph | $CH_3$ | $OCH_3$ | " | 22 | 141-2 | 3120,1635<br>1595 | $C_{22}H_{20}N_2O_{2.2}$ | 344,4 | 76,72<br>76,7 | 5,85<br>5,9 | 8,13<br>8,2 | |
| 9 | H | COPh | H | $CH_3$ | " | 22 | 187-9 | 3100,1625<br>1595 | $C_{21}H_{18}N_2O$ | 314,4 | 80,23<br>80,4 | 5,77<br>5,8 | 8,91<br>8,9 | |
| 10 | H | COPh | $C_2H_5$ | $OCH_3$ | " | 18 | 145-6 | 3120,1630<br>1570 | $C_{23}H_{22}N_2O_2$ | 358,4 | 77,07<br>77,1 | 6,19<br>6,2 | 7,81<br>7,8 | |

Les valeurs ci-dessus sont les concentrations nanomolaires du produit testé qui inhibent dans une proportion de 50 % la liaison spécifique de 0,6 nanomole ($^3H$) flunitrazépam tant de préparations de membranes de cerveaux antérieurs de rats ($CI_{50}$) nanomoles.

| PRODUIT DE L'EXEMPLE | $CI_{50}$ nM |
|---|---|
| 1 | 5000 |
| 2 | 3200 |
| 3 | 975 |
| 4 | 430 |
| 5 | 8000 |
| 6 | 395 |
| 7 | 120 |
| 8 | 7 |
| 9 | 250 |
| 10 | 135 |

Exemple de compositions pharmaceutiques

On a préparé des composés correspondant à la formule suivante :

| | |
|---|---|
| (7-éthyl 5-méthoxy 4-méthylimidazo [1,2-a] quinoléin-2-yl) phénylméthanone | 20 mg |
| Excipient q.s. pour un comprimé terminé à | 150 mg |

(Détail de l'excipient : lactose, amidon, talc, stéarate de magnésium).

Exemple B

On a préparé des comprimés correspondant à la formule suivante :

| | |
|---|---|
| (4,7-diéthyl 5-méthoxyimidazo [1,2-a] quinoléin-2-yl) phénylméthanone | 20 mg |
| Excipient q.s. pour un comprimé terminé à | 150 mg |

(Détail de l'excipient : lactose, amidon, talc, stéarate de magnésium).

**Revendications** (pour les Etats contractants : BE, CH, DE, FR, IT, LI, LU, NL, SE)

1. Imidazo [1,2-a] quinoléines et leurs sels, caractérisées en ce qu'elles répondent à la formule générale (I) :

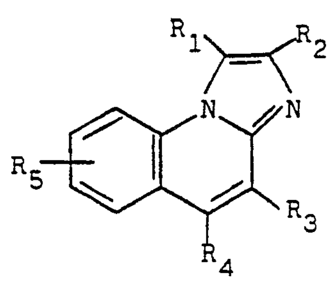

(I)

dans laquelle $R_1$ représente un atome d'hydrogène ou un atome de chlore ou de brome, $R_2$ représente un radical benzoyle, un radical

$$-\underset{\phi}{CH}-O-COR_6$$

dans lequel $R_6$ représente un radical alcoyle renfermant de 1 à 5 atomes de carbone, un radical :

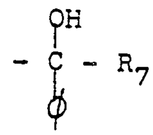

dans lequel $R_7$ représente un radical alcoyle renfermant de 1 à 5 atomes de carbone, ou un radical

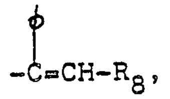

dans lequel $R_8$ représente un atome d'hydrogène ou un radical alcoyle renfermant de 1 à 4 atomes de carbone, le symbole $\phi$ représentant un radical phényle, $R_3$ représente un atome d'hydrogène ou un radical alcoyle renfermant de 1 à 5 atomes de carbone, $R_4$ représente un radical alcoxy renfermant de 1 à 8 atomes de carbone, un radical hydroxy, un radical acyloxy renfermant de 2 à 8 atomes de carbone, un radical alcoyle renfermant de 1 à 8 atomes de carbone, $R_5$ représente un radical alcoyle renfermant de 1 à 8 atomes de carbone, étant entendu que $R_1$ et $R_3$ ne peuvent représenter un atome d'hydrogène lorsque $R_2$ représente un radical benzoyle et $R_4$ représente un radical alcoxy renfermant de 1 à 8 atomes de carbone.

2. Dérivés répondant à la formule (I) de la revendication 1 ainsi que leurs sels, caractérisés en ce que dans ladite formule (I), $R_3$ représente un atome d'hydrogène ou un radical méthyle ou éthyle, $R_4$ représente un radical méthoxy, hydroxy acétoxy ou méthyle, $R_5$ représente un radical méthyle ou éthyle $R_1$ et $R_2$ ayant la signification déjà indiquée.

3. Dérivés répondant à la formule (I) de la revendication 1 ainsi que leurs sels, caractérisés en ce que dans ladite formule (I), $R_1$ représente un atome d'hydrogène, $R_2$ représente un radical benzoyle, un radical 1-phényl vinyle, $R_3$ représente un atome d'hydrogène, un radical méthyle ou éthyle, $R_4$ représente un radical méthoxy, hydroxy, acétoxy ou méthyle $R_5$ représente un radical éthyle.

4. Dérivés répondant à la formule (I) de la revendication 1, ainsi que leurs sels, caractérisés en ce que dans ladite formule (I), $R_1$ représente un atome d'hydrogène, $R_2$ représente un radical benzoyle, $R_3$ représente un radical méthyle ou éthyle, $R_4$ représente un radical méthoxy, $R_5$ représente un radical éthyle.

5. La (7-éthyl 5-méthoxy 4-méthyl [imidazo] 1,2-a [quinoléin-2-yl]) phényl méthanone et ses sels.

6. La (4,7-diéthyl 5-méthoxy [imidazo] 1,2-a [quinoléin-2-yl]) phényl méthanone et ses sels.

7. Procédé de préparation des produits de formule (I) dans laquelle $R_1$ représente un atome d'hydrogène, $R_2$ représente un radical benzoyle, $R_4$ représente un radical $R'_4$ lequel représente un radical alcoxy renfermant de 1 à 8 atomes de carbone ou un radical alcoyle renfermant de 1 à 8 atomes de carbone, $R_3$ et $R_5$ ont la signification déjà indiquée, ainsi que de leurs sels, caractérisé en ce que l'on fait réagir un produit de formule (II) :

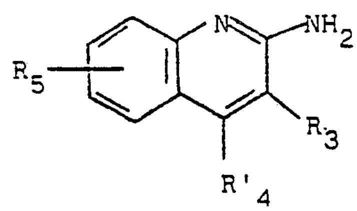

(II)

dans laquelle $R_3$, $R'_4$ et $R_5$ ont la signification déjà indiquée, avec un produit de formule (III) :

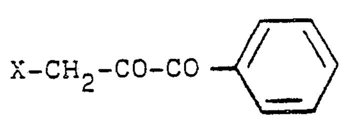

(III)

dans laquelle X représente un atome d'halogène, pour obtenir un produit de formule (IV) :

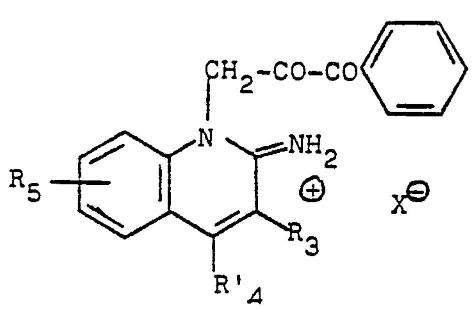

(IV)

dans laquelle $R_3$, $R'_4$ et $R_5$ ont la signification déjà indiquée, puis cyclise le produit de formule (IV) obtenu pour former le produit de formule ($I_A$) :

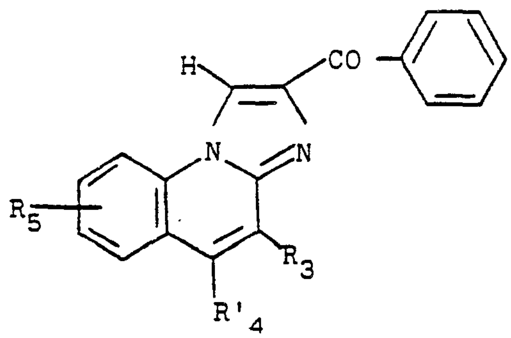

($I_A$)

dans laquelle $R_3$, $R'_4$ et $R_5$ ont la signification précitée, que l'on salifie, le cas échéant.

8. Procédé selon la revendication 7, caractérisé en ce que :

a) la réaction du produit de formule (II) avec le produit de formule (III) est effectuée au sein d'un solvant organique tel que le diméthoxyméthane ;

b) la cyclisation du produit de formule (IV) est effectuée par chauffage au sein d'un solvant organique tel que l'éthanol ;

c) la réaction du produit de formule (II) avec le produit de formule (III) est poursuivie jusqu'au produit de formule ($I_A$) sans isoler le produit de formule (IV).

9. Procédé de préparation des produits de formule (I) dans laquelle $R_1$ représente un atome de chlore ou de brome, $R_2$ représente un radical benzoyle, ainsi que de leurs sels, caractérisé en ce que l'on halogène un produit correspondant dans lequel $R_1$ représente un atome d'hydrogène, pour obtenir un produit de formule ($I_B$) :

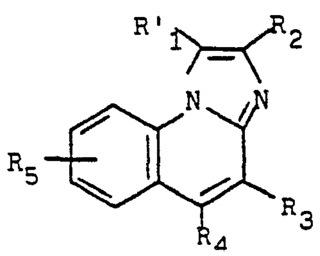

($I_B$)

dans laquelle $R'_1$ représente un atome de chlore ou de brome, $R_2$, $R_3$, $R_4$ et $R_5$ ayant la signification déjà indiquée, que l'on isole, et si désiré, salifie.

10. Procédé selon la revendication 9, caractérisé en ce que :

a) l'halogénation est effectuée au moyen du N-bromosuccinimide lorsque l'on désire préparer un produit de formule (I) dans laquelle $R_1$ représente un atome de brome,

b) l'halogénation est effectuée au moyen du N-chlorosuccinimide lorsque l'on désire préparer un produit de formule (I) dans laquelle $R_1$ représente un atome de chlore.

11. Procédé de préparation des produits de formule (I) dans laquelle $R_4$ représente un radical $R'_4$, $R_1$, $R_3$, $R'_4$ et $R_5$ ont la signification déjà indiquée et $R_2$ représente un radical benzoyle ainsi que de leurs sels, caractérisé en ce que l'on réduit un produit de formule (V) :

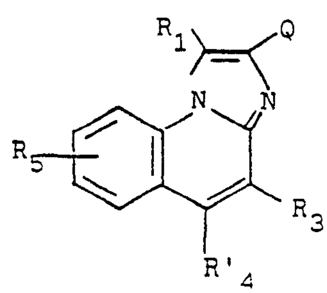

(V)

dans laquelle $R_1$, $R_3$, $R'_4$ et $R_5$ ont la signification déjà indiquée et Q représente un groupement alcoxy carbonyl renfermant de 2 à 4 atomes de carbone ou aralcoxycarbonyl renfermant de 8 à 12 atomes de carbone, pour obtenir un produit de formule (VI) :

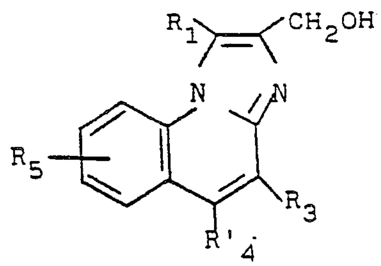

(VI)

dans laquelle $R_1$, $R_3$, $R'_4$ et $R_5$ ont la signification déjà indiquée, que l'on oxyde pour obtenir un produit de formule (VII) :

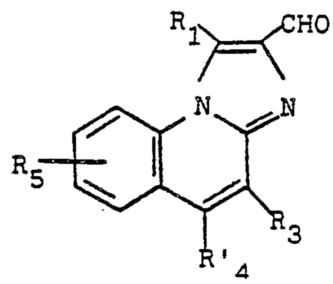

(VII)

dans laquelle $R_1$, $R_3$, $R'_4$ et $R_5$ ont la signification déjà indiquée, que l'on soumet à l'action d'un réactif de Grignard formé à partir d'un produit de formule (VIII) :

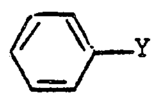

(VIII)

dans laquelle Y représente un atome de chlore, d'iode ou de brome ou à l'action du phényllithium, pour obtenir un produit de formule (IX) :

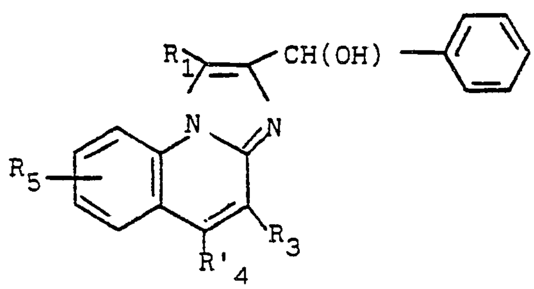

(IX)

dans laquelle $R_1$, $R_3$, $R'_4$ et $R_5$ ont la signification déjà indiquée, que l'on oxyde pour obtenir un produit de formule ($I_C$) :

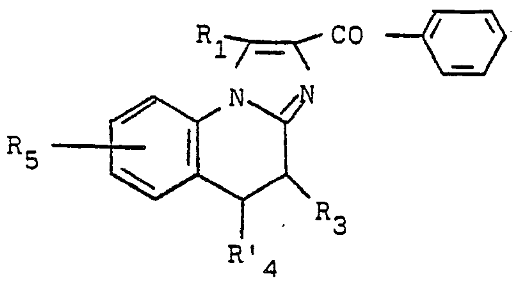

($I_C$)

dans laquelle $R_1$, $R_3$, $R'_4$ et $R_5$ ont la signification déjà indiquée, que l'on isole et, si désiré, salifie.

12. Procédé selon la revendication 11, caractérisé en ce que :

a) la réduction du produit de formule (V) est effectuée par l'hydrure de lithium-aluminium, le mélange borohydrure de sodium-chlorure d'aluminium ou le borohydrure de lithium ;

b) l'oxydation du produit de formule (VI) est effectuée à l'aide de dioxyde de manganèse ou encore d'acide nitrique, de chlorure ferrique, de mélange oxyde de chrome-pyridine ou encore par la méthode d'Oppenauer ou par déshydrogénation sur un catalyseur à base de cuivre ;

c) la réaction du produit de formule (VII) avec le réactif de Grignard ou avec le phényllithium est effectuée au sein du tétrahydrofuranne ;

d) l'oxydation du produit de formule (IX) est effectuée par l'une des méthodes indiquées ci-dessus.

13. Procédé de préparation des produits de formule (I) dans laquelle $R_2$ représente un radical :

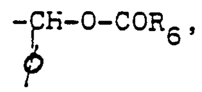

$R_1$, $R_3$, $R_5$ étant définis comme précédemment et $R_4$ étant différent de l'hydroxy ainsi que de leurs sels caractérisé en ce que l'on réduit un produit de formule :

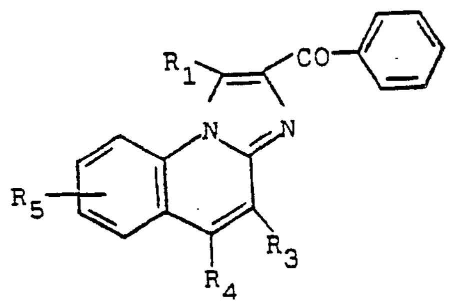

(A)

dans laquelle $R_1$, $R_3$, $R_4$ et $R_5$ sont définis comme précédemment, pour obtenir un produit de formule (X) :

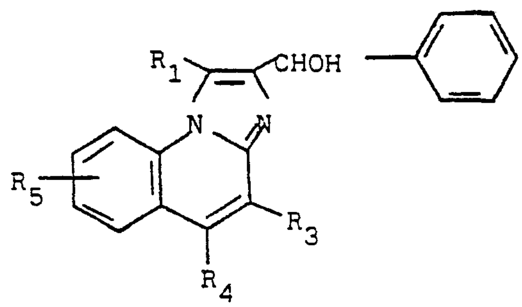

(X)

dans laquelle $R_1$, $R_3$, $R_4$ et $R_5$ sont définis comme précédemment, que l'on soumet à une réaction d'acylation, pour obtenir un produit de formule ($I_D$) :

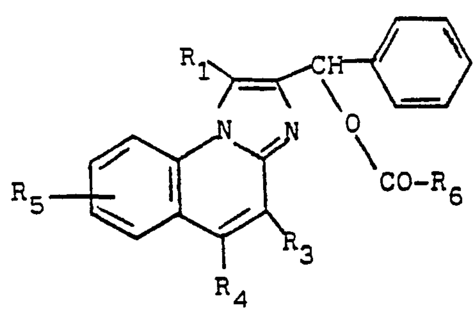

($I_D$)

dans laquelle $R_1$, $R_3$, $R_4$ et $R_5$ ont la signification déjà indiquée que l'on isole et, si désiré, salifie.

14. Procédé selon la revendication 13, caractérisé en ce que :

a) la réduction du produit de formule (A) est effectuée au moyen du borohydrure de sodium au sein d'un solvant organique tel que l'éthanol,

b) l'acylation du produit de formule (X) est effectuée au moyen d'un halogénure d'acyle, notamment un chlorure ou un bromure, au sein de la pyridine.

15. Procédé de préparation des produits de formule (I) dans laquelle $R_2$ représente un radical :

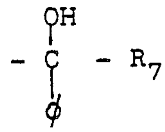

ou un radical

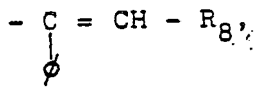

$R_1$, $R_3$, $R_5$, $R_7$ et $R_8$ étant définis comme précédemment et $R_4$ est différent d'acyloxy ainsi que de leurs sels, caractérisé en ce que l'on fait réagir un produit de formule (A) tel que défini précédemment, avec un réactif de Grignard formé à partir d'un produit de formule (XI) :

$$Y—R_7 \qquad (XI)$$

ou avec un réactif de formule $R_7$—Li dans lesquelles Y et $R_7$ sont définis comme précédemment, pour obtenir un produit de formule $I_E$ :

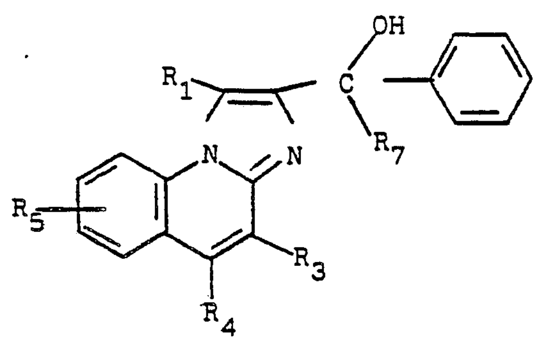

($I_E$)

dans laquelle $R_1$, $R_3$, $R_4$, $R_5$ et $R_7$ ont la signification déjà indiquée et que,

soit on isole et, si désiré, salifie le produit de formule ($I_E$) ainsi obtenu,

soit on déshydrate ce dernier dans le cas où $R_7$ représente un radical $—CH_2R_8$, $R_8$ étant défini comme précédemment, pour obtenir un produit de formule ($I_F$) :

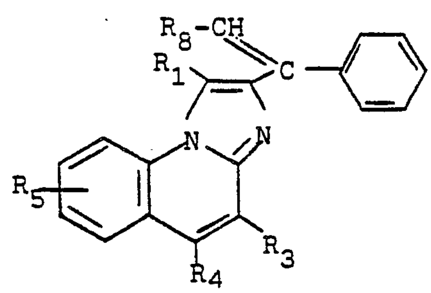

($I_F$)

dans laquelle $R_1$, $R_3$, $R_4$, $R_5$ et $R_8$ ont la signification déjà indiquée, que l'on isole et, si désiré, salifie.

16. Procédé selon la revendication 15, caractérisé en ce que :

a) la réaction du produit de formule (A) avec le produit de formule (XI) ou avec le réactif de formule $R_7$-Li est effectuée au sein de l'éther anhydre.

b) la déshydratation du produit de formule ($I_D$) est effectuée au moyen d'acide chlorhydrique concentré.

17. Procédé de préparation des produits de formule (I) dans laquelle $R_1$, $R_3$ et $R_5$ sont définis comme précédemment, $R_2$ représente un radical benzoyle ou un groupement

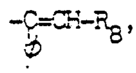

$R_8$ étant défini comme précédemment et $R_4$ représente un radical hydroxy ou un radical acyloxy renfermant de 2 à 8 atomes de carbone, ainsi que de leurs sels, caractérisé en ce que l'on hydrolyse un produit correspondant dans lequel $R_4$ représente un radical alcoxy renfermant de 1 à 8 atomes de carbone, pour obtenir un produit de formule ($I_G$) :

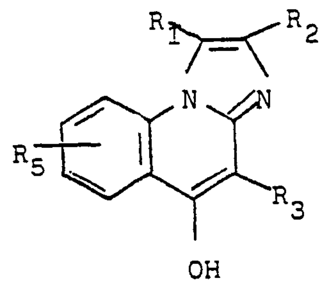

($I_G$)

dans laquelle $R_1$, $R_2$, $R_3$ et $R_5$ ont la signification déjà indiquée et que,
soit on isole et, si désiré, salifie le produit de formule ($I_G$) ainsi obtenu,
soit on fait réagir ce dernier avec un halogénure de formule (XII) :

$$Hal—R_9 \qquad (XII)$$

dans laquelle $R_9$ représente un radical acyle renfermant de 2 à 8 atomes de carbone et Hal représente un atome de chlore ou de brome, ou avec un anhydride d'acide de formule (XIII) :

$$(R_9)_2O \qquad (XIII)$$

dans laquelle $R_9$ a la signification déjà indiquée, pour obtenir un produit de formule ($I_H$) :

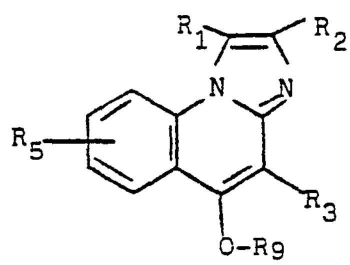

($I_H$)

dans laquelle $R_1$, $R_2$, $R_3$, $R_9$ et $R_5$ ont la signification déjà indiquée, que l'on isole et, si désiré, salifie.

18. Procédé selon la revendication 17, caractérisé en ce que :

a) l'hydrolyse du produit dans lequel $R_4$ représente un radical alcoxy est effectuée au moyen de l'acide bromhydrique par chauffage au reflux du milieu réactionnel pendant 30 heures en présence d'eau ;

b) la réaction du produit de formule ($I_G$) avec l'halogénure de formule (XII) ou l'anhydride d'acide de formule (XIII) est effectuée au sein de la triéthylamine.

19. Médicaments, caractérisés en ce qu'ils sont constitués par les nouvelles imidazo [1,2-a] quinoléines telles que définies à la revendication 1 ainsi que par leurs sels d'addition avec les acides pharmaceutiquement acceptables.

20. Médicaments, caractérisés en ce qu'ils sont constitués par les nouvelles imidazo [1,2-a] quinoléines telles que définies à la revendication 2, 3 ou 4, ainsi que par leurs sels d'addition avec les acides pharmaceutiquement acceptables.

21. Médicaments, caractérisés en ce qu'ils sont constitués par les dérivés tels que définis à la revendication 5 ou 6, ainsi que par les sels d'addition avec les acides pharmaceutiquement acceptables desdits dérivés.

22. Compositions pharmaceutiques, caractérisées en ce qu'elles renferment à titre de principe actif, l'un au moins des médicaments tels que définis à l'une quelconque des revendications 19, 20 ou 21.

**Revendications** (pour l'Etat contractant AT)

1. Procédé de préparation de nouvelles imidazo [1,2-a] quinoléines et de leurs sels, répondant à la formule générale (I) :

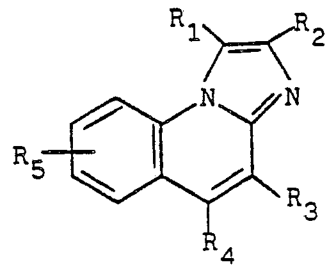

(I)

dans laquelle $R_1$ représente un atome d'hydrogène ou un atome de chlore ou de brome, $R_2$ représente un radical benzoyle, un radical

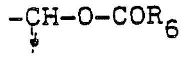

dans lequel $R_6$ représente un radical alcoyle renfermant de 1 à 5 atomes de carbone, un radical :

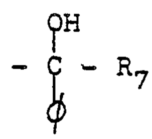

dans lequel $R_7$ représente un radical alcoyle renfermant de 1 à 5 atomes de carbone, ou un radical

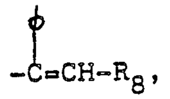

dans lequel $R_8$ représente un atome d'hydrogène ou un radical alcoyle renfermant de 1 à 4 atomes de carbone, le symbole $\phi$ représentant un radical phényle, $R_3$ représente un atome d'hydrogène ou un radical alcoyle renfermant de 1 à 5 atomes de carbone, $R_4$ représente un radical alcoxy renfermant de 1 à 8 atomes de carbone, un radical hydroxy, un radical acyloxy renfermant de 2 à 8 atomes de carbone, un radical alcoyle renfermant de 1 à 8 atomes de carbone, $R_5$ représente un radical alcoyle renfermant de 1 à 8 atomes de carbone, étant entendu que $R_1$ et $R_3$ ne peuvent représenter un atome d'hydrogène lorsque $R_2$ représente un radical benzoyle et $R_4$ représente un radical alcoxy renfermant de 1 à 8 atomes de carbone, caractérisé en ce que :

pour la préparation des produits de formule (I) dans laquelle $R_1$ représente un atome d'hydrogène, $R_2$ représente un radical benzoyle, $R_4$ représente un radical $R'_4$, lequel représente un radical alcoxy renfermant de 1 à 8 atomes de carbone ou un radical alcoyle renfermant de 1 à 8 atomes de carbone, $R_3$ et $R_5$ ont la signification déjà indiquée, ainsi que leurs sels, l'on fait réagir un produit de formule (II) :

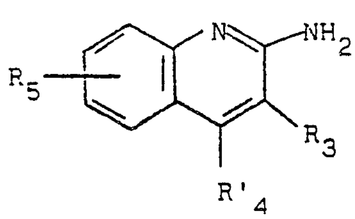

(II)

dans laquelle $R_3$, $R'_4$ et $R_5$ ont la signification déjà indiquée, avec un produit de formule (III) :

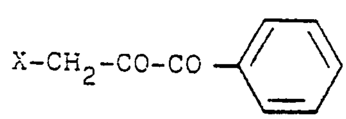

(III)

dans laquelle X représente un atome d'halogène, pour obtenir un produit de formule (IV) :

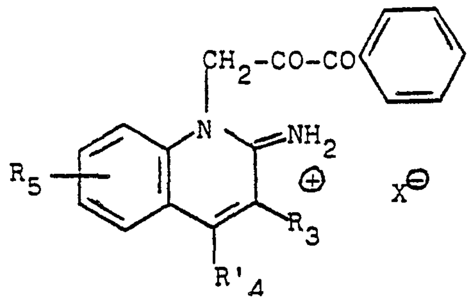

(IV)

dans laquelle $R_3$, $R'_4$ et $R_5$ ont la signification déjà indiquée, puis cyclise le produit de formule (IV) obtenu pour former le produit de formule ($I_A$) :

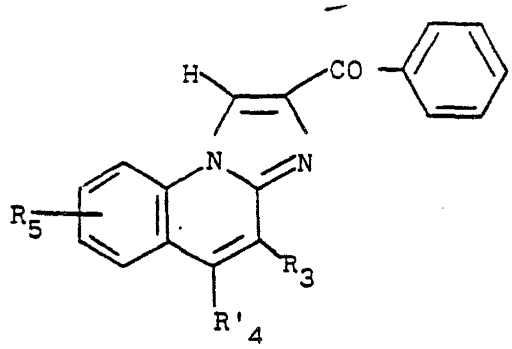

($I_A$)

dans laquelle $R_3$, $R'_4$ et $R_5$ ont la signification précitée, que l'on salifie, le cas échéant ;

pour la préparation des produits de formule (I) dans laquelle $R_1$ représente un atome de chlore ou de brome, $R_2$ représente un radical benzoyle, ainsi que de leurs sels, l'on halogène un produit correspondant dans lequel $R_1$ représente un atome d'hydrogène pour obtenir un produit de formule ($I_B$) :

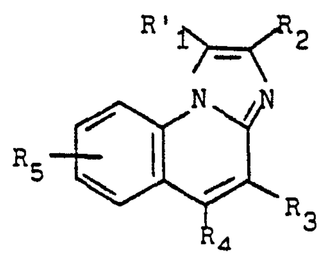

($I_B$)

dans laquelle $R'_1$ représente un atome de chlore ou de brome, $R_2$, $R_3$, $R_4$ et $R_5$ ayant la signification déjà indiquée, que l'on isole, et, si désiré, salifie.

pour la préparation des produits de formule (I) dans laquelle $R_4$ représente un radical $R'_4$, $R_1$, $R_3$, $R'_4$ et $R_5$ ont la signification déjà indiquée, et $R_2$ représente un radical benzoyle, ainsi que de leurs sels, l'on réduit un produit de formule (V) :

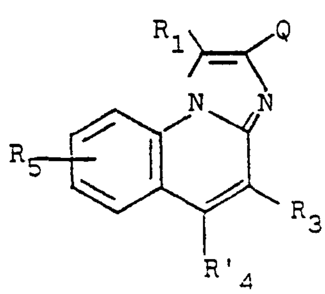

(V)

dans laquelle $R_1$, $R_3$, $R'_4$ et $R_5$ ont la signification déjà indiquée et Q représente un groupement alcoxy carbonyl renfermant de 2 à 4 atomes de carbone ou aralcoxycarbonyl renfermant de 8 à 12 atomes de carbone, pour obtenir un produit de formule (VI) :

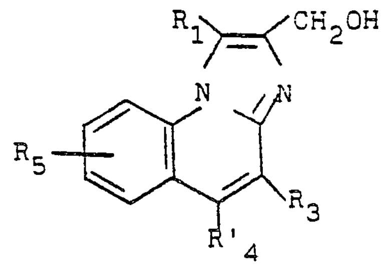

(VI)

dans laquelle $R_1$, $R_3$, $R'_4$ et $R_5$ ont la signification déjà indiquée, que l'on oxyde pour obtenir un produit de formule (VII) :

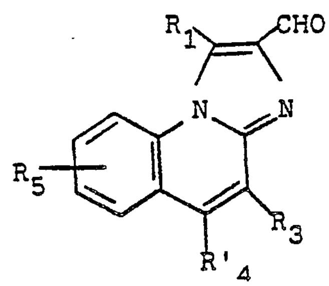

(VII)

dans laquelle $R_1$, $R_3$, $R'_4$ et $R_5$ ont la signification déjà indiquée, que l'on soumet à l'action d'un réactif de Grignard formé à partir d'un produit de formule (VIII) :

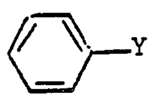

(VIII)

dans laquelle Y représente un atome de chlore, d'iode ou de brome ou à l'action du phényllithium, pour obtenir un produit de formule (IX) :

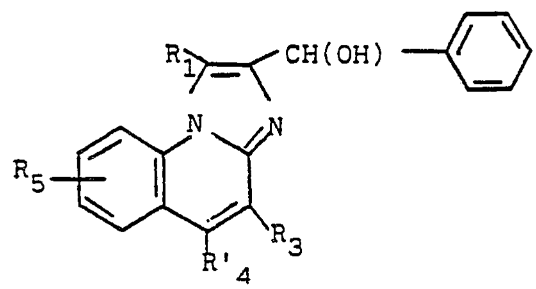

(IX)

dans laquelle $R_1$, $R_3$, $R'_4$ et $R_5$ ont la signification déjà indiquée, que l'on oxyde pour obtenir un produit de formule ($I_C$) :

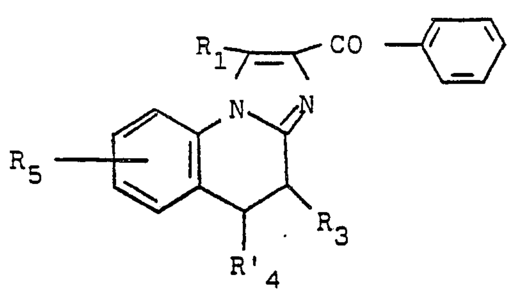

($I_C$)

dans laquelle $R_1$, $R_3$, $R'_4$ et $R_5$ ont la signification déjà indiquée, que l'on isole et, si désiré, salifie, pour la préparation des produits de formule (I) dans laquelle $R_2$ représente un radical

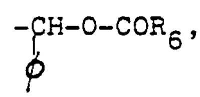

$R_1$, $R_3$, $R_5$ étant définis comme précédemment et $R_4$ étant différent d'hydroxy ainsi que de leurs sels, l'on réduit un produit de formule :

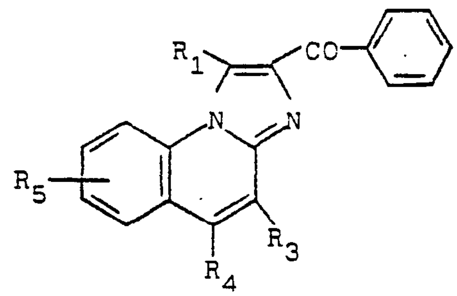

(A)

dans laquelle $R_1$, $R_3$, $R_4$ et $R_5$ sont définis comme précédemment, pour obtenir un produit de formule (X) :

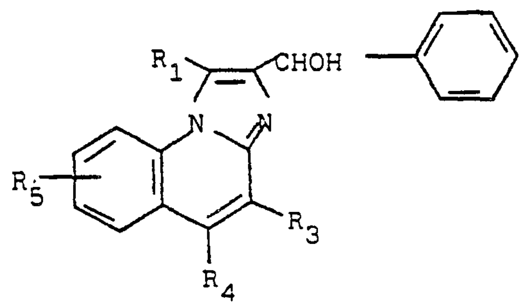

(X)

dans laquelle $R_1$, $R_3$, $R_4$ et $R_5$ sont définis comme précédemment, que l'on soumet à une réaction d'acylation, pour obtenir un produit de formule ($I_D$) :

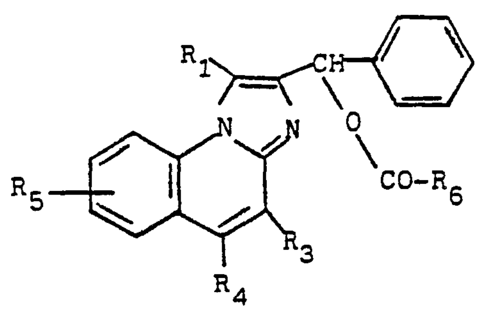

($I_D$)

dans laquelle $R_1$, $R_3$, $R_4$ et $R_5$ ont la signification déjà indiquée que l'on isole et, si désiré, salifie ;

pour la préparation des produits de formule (I) dans laquelle $R_2$ représente un radical :

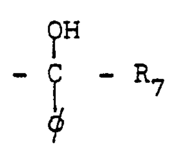

ou un radical :

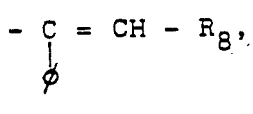

$R_1$, $R_3$, $R_5$, $R_7$ et $R_8$ étant définis comme précédemment et $R_4$ est différent d'acyloxy ainsi que de leurs sels, l'on fait réagir un produit de formule (A) tel que défini précédemment, avec un réactif de Grignard formé à partir d'un produit de formule (XI) :

$$Y—R_7 \qquad (XI)$$

ou avec un réactif de formule : $R_7$—Li dans laquelle Y et $R_7$ sont définis comme précédemment, pour obtenir un produit de formule ($I_E$) :

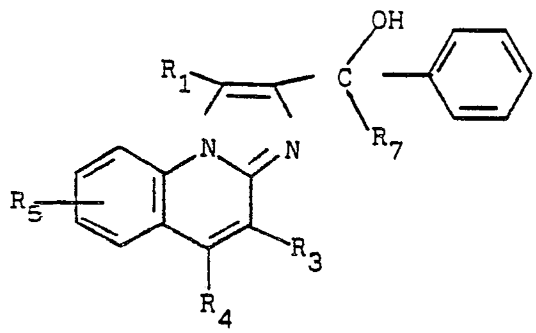

($I_E$)

dans laquelle $R_1$, $R_3$, $R_4$, $R_5$ et $R_7$ ont la signification déjà indiquée et que,

soit on isole et, si désiré, salifie le produit de formule ($I_E$) ainsi obtenu,

soit on déshydrate ce dernier dans le cas où $R_7$ représente un radical $—CH_2R_8$, $R_8$ étant défini comme précédemment, pour obtenir un produit de formule ($I_F$) :

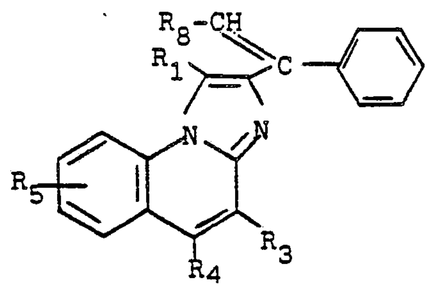

($I_F$)

dans laquelle $R_1$, $R_3$, $R_4$, $R_5$ et $R_8$ ont la signification déjà indiquée, que l'on isole et, si désiré, salifie ;

pour la préparation des produits de formule (I) dans laquelle $R_1$, $R_3$ et $R_5$ sont définis comme précédemment, $R_2$ représente un radical benzoyle ou un groupement :

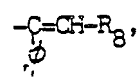

$R_8$ étant défini comme précédemment et $R_4$ représente un radical hydroxy ou un radical acyloxy renfermant de 2 à 8 atomes de carbone, ainsi que de leurs sels, l'on hydrolyse un produit correspondant dans lequel $R_4$ représente un radical alcoxy renfermant de 1 à 8 atomes de carbone, pour obtenir un produit de formule ($I_G$) :

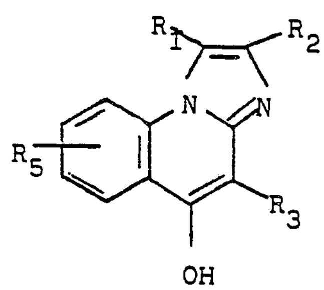

($I_G$)

dans laquelle $R_1$, $R_2$, $R_3$ et $R_5$ ont la signification déjà indiquée et que,

soit on isole et, si désiré, salifie le produit de formule ($I_G$) ainsi obtenu,

soit on fait réagir ce dernier avec un halogénure de formule (XII) :

$$Hal—R_9 \qquad (XII)$$

dans laquelle $R_9$ représente un radical acyle renfermant de 2 à 8 atomes de carbone et Hal représente un atome de chlore ou de brome, ou avec un anhydride d'acide de formule (XIII) :

$$(R_9)_2O \qquad (XIII)$$

dans laquelle $R_9$ a la signification déjà indiquée, pour obtenir un produit de formule ($I_H$) :

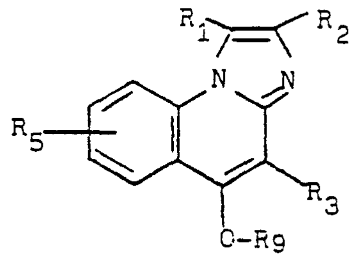

($I_H$)

dans laquelle $R_1$, $R_2$, $R_3$, $R_9$ et $R_5$ ont la signification déjà indiquée, que l'on isole, et si désiré, salifie.

2. Procédé selon la revendication 1, pour la préparation des produits de formule (I) dans laquelle $R_1$ représente un atome d'hydrogène, $R_2$ représente un radical benzoyle, $R_4$ représente un radical $R'_4$, lequel représente un radical alcoxy renfermant de 1 à 8 atomes de carbone ou un radical alcoyle renfermant de 1 à 8 atomes de carbone, $R_3$ et $R_5$ ont la signification déjà indiquée, ainsi que de leurs sels, caractérisés en ce que l'on fait réagir un produit de formule (II) :

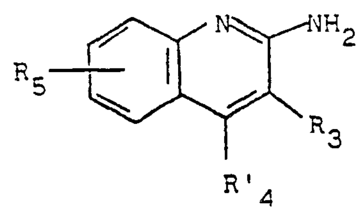

(II)

dans laquelle $R_3$, $R'_4$ et $R_5$ ont la signification déjà indiquée, avec un produit de formule (III) :

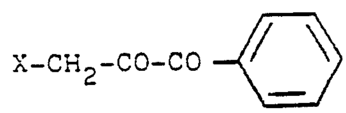

(III)

dans laquelle X représente un atome d'halogène, pour obtenir un produit de formule (IV) :

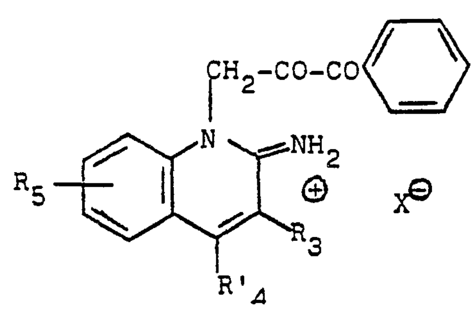

(IV)

dans laquelle $R_3$, $R'_4$, $R_5$ ont la signification déjà indiquée, puis cyclise le produit de formule (IV) obtenu pour former le produit de formule ($I_A$) :

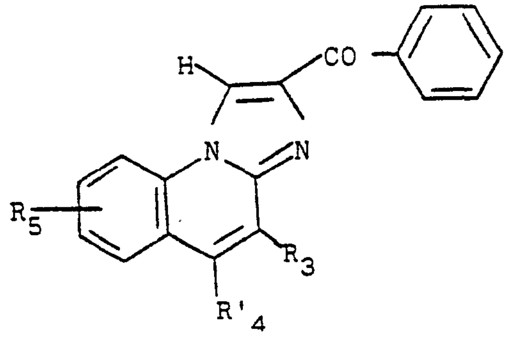

($I_A$)

dans laquelle $R_3$, $R'_4$ et $R_5$ ont la signification précitée, que l'on salifie, le cas échéant.

3. Procédé selon la revendication 2, caractérisé en ce que :

a) la réaction du produit de formule (II) avec le produit de formule (III) est effectuée au sein d'un solvant organique tel que le diméthoxyméthane ;

b) la cyclisation du produit de formule (IV) est effectuée par chauffage au sein d'un solvant organique tel que l'éthanol ;

c) la réaction du produit de formule (II) avec le produit de formule (III) est poursuivie jusqu'au produit de formule ($I_A$) sans isoler le produit de formule (IV).

4. Procédé selon la revendication 1, pour la préparation des produits de formule (I) dans laquelle $R_1$ représente un atome de chlore ou de brome, $R_2$ représente un radical benzoyle, ainsi que de leurs sels, caractérisé en ce que l'on halogène un produit correspondant dans lequel $R_1$ représente un atome d'hydrogène, pour obtenir un produit de formule ($I_B$) :

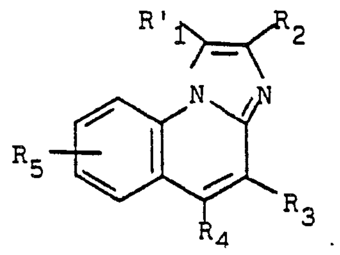

($I_B$)

dans laquelle $R'_1$ représente un atome de chlore ou de brome, $R_2$, $R_3$, $R_4$ et $R_5$ ayant la signification déjà indiquée, que l'on isole, et si désiré, salifie.

5. Procédé selon la revendication 4, caractérisé en ce que :

a) l'halogénation est effectuée au moyen du N-bromosuccinimide lorsque l'on désire préparer un produit de formule (I) dans laquelle $R_1$ représente un atome de brome,

b) l'halogénation est effectuée au moyen du N-chlorosuccinimide lorsque l'on désire préparer un produit de formule (I) dans laquelle $R_1$ représente un atome de chlore.

6. Procédé selon la revendication 1, pour la préparation des produits de formule (I) dans laquelle $R_4$ représente un radical $R'_4$, $R_1$, $R_3$, $R'_4$ et $R_5$ ont la signification déjà indiquée et $R_2$ représente un radical benzoyle, ainsi que de leurs sels, caractérisé en ce que l'on réduit un produit de formule (V) :

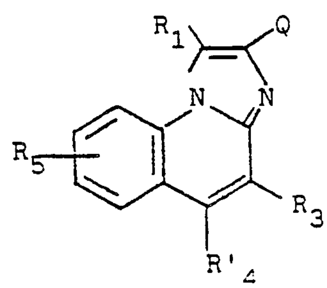

(V)

dans laquelle $R_1$, $R_3$, $R'_4$ et $R_5$ ont la signification déjà indiquée et Q représente un groupement alcoxy carbonyl renfermant de 2 à 4 atomes de carbone ou aralcoxycarbonyl renfermant de 8 à 12 atomes de carbone, pour obtenir un produit de formule (VI) :

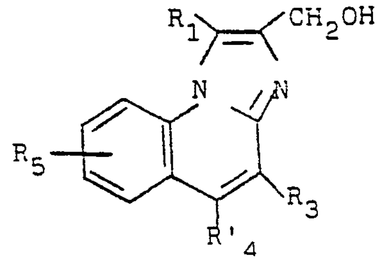

(VI)

dans laquelle $R_1$, $R_3$, $R'_4$ et $R_5$ ont la signification déjà indiquée, que l'on oxyde pour obtenir un produit de formule (VII) ;

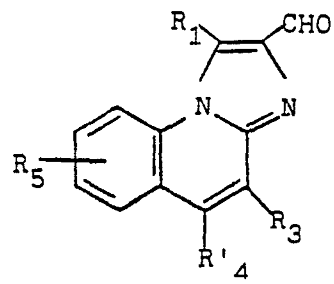

(VII)

dans laquelle $R_1$, $R_3$, $R'_4$ et $R_5$ ont la signification déjà indiquée, que l'on soumet à l'action d'un réactif de Grignard formé à partir d'un produit de formule (VIII) :

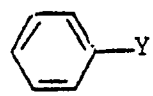

(VIII)

dans laquelle Y représente un atome de chlore, d'iode ou de brome ou à l'action du phényllithium, pour obtenir un produit de formule (IX) :

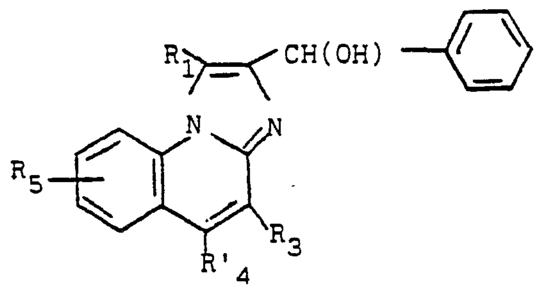

(IX)

dans laquelle $R_1$, $R_3$, $R'_4$ et $R_5$ ont la signification déjà indiquée, que l'on oxyde pour obtenir un produit de formule ($I_C$) :

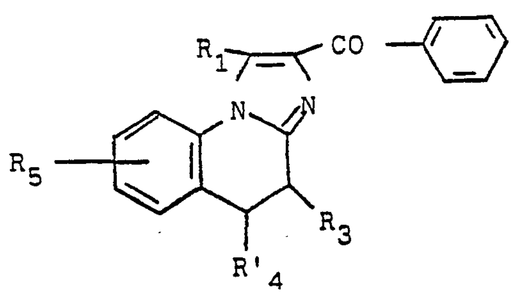

($I_C$)

dans laquelle $R_1$, $R_3$, $R'_4$ et $R_5$ ont la signification déjà indiquée, que l'on isole et, si désiré, salifie.

7. Procédé selon la revendication 6, caractérisé en ce que :

a) la réduction du produit de formule (V) est effectuée par l'hydrure de lithium-aluminium, le mélange borohydrure de sodium-chlorure d'aluminium ou le borohydrure de lithium ;

b) l'oxydation du produit de formule (VI) est effectuée à l'aide de dioxyde de manganèse ou encore d'acide nitrique, de chlorure ferrique, de mélange oxyde de chrome-pyridine, ou encore par la méthode d'Oppenauer ou par déshydrogénation sur un catalyseur à base de cuivre ;

c) la réaction du produit de formule (VII) avec le réactif de Grignard ou avec le phényl lithium est effectuée au sein du tétrahydrofuranne ;

d) l'oxydation du produit de formule (IX) est effectuée par l'une des méthodes indiquées ci-dessus.

8. Procédé selon la revendication 1, pour la préparation des produits de formule (I) dans laquelle $R_2$ représente un radical :

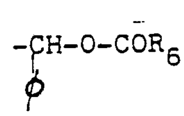,

$R_1$, $R_3$, $R_5$ étant définis comme précédemment et $R_4$ étant différent d'hydroxy ainsi que de leurs sels, caractérisé en ce que l'on réduit un produit de formule :

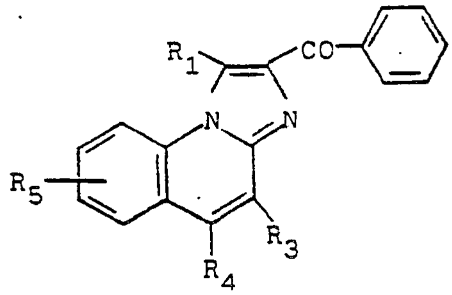

(A)

dans laquelle $R_1$, $R_3$, $R_4$ et $R_5$ sont définis comme précédemment, pour obtenir un produit de formule (X) :

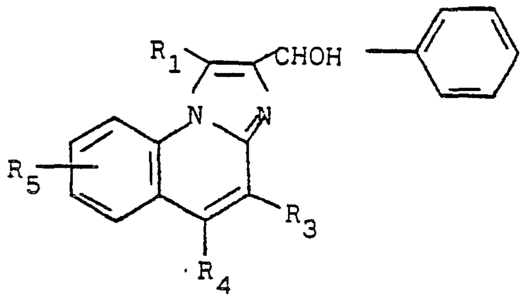

(X)

dans laquelle $R_1$, $R_3$, $R_4$ et $R_5$ sont définis comme précédemment, que l'on soumet à une réaction d'acylation, pour obtenir un produit de formule ($I_D$) :

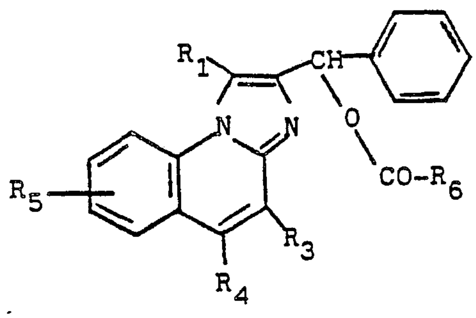

($I_D$)

dans laquelle $R_1$, $R_3$, $R_4$ et $R_5$ ont la signification déjà indiquée que l'on isole et, si désiré, salifie.

9. Procédé selon la revendication 8, caractérisé en ce que :

a) la réduction du produit de formule (A) est effectuée au moyen du borohydrure de sodium au sein d'un solvant organique tel que l'éthanol,

b) l'acylation du produit de formule (X) est effectuée au moyen d'un halogénure d'acyle, notamment un chlorure ou un bromure au sein de la pyridine.

10. Procédé selon la revendication 1, pour la préparation des produits de formule (I) dans laquelle $R_2$ représente un radical :

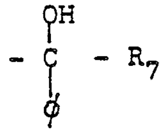

ou un radical

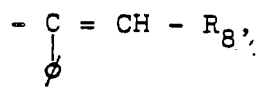

$R_1$, $R_3$, $R_5$, $R_7$ et $R_8$ étant définis comme précédemment et $R_4$ est différent d'acyloxy ainsi que de leurs sels, caractérisé en ce que l'on fait réagir un produit de formule (A) tel que défini précédemment avec un réactif de Grignard formé à partir d'un produit de formule (XI) :

$Y—R_7$ (XI)

ou avec un réactif de formule : $R_7$—Li dans laquelle Y et $R_7$ sont définis comme précédemment, pour obtenir un produit de formule ($I_E$) :

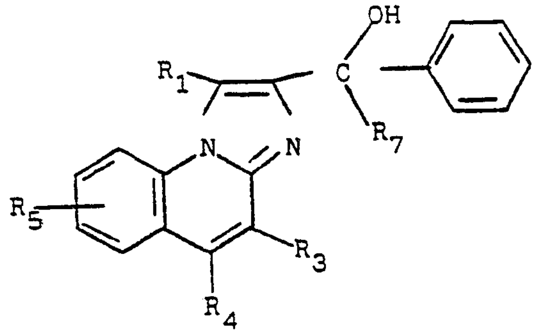

($I_E$)

dans laquelle $R_1$, $R_3$, $R_4$, $R_5$ et $R_7$ ont la signification déjà indiquée et que,

soit on isole et, si désiré, salifie le produit de formule ($I_E$) ainsi obtenu,

soit on déshydrate ce dernier dans le cas où $R_7$ représente un radical $—CH_2R_8$, $R_8$ étant défini comme précédemment, pour obtenir un produit de formule ($I_F$) :

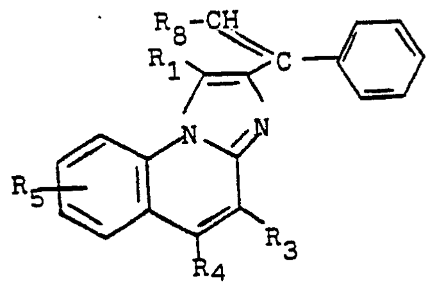

($I_F$)

dans laquelle $R_1$, $R_3$, $R_4$, $R_5$ et $R_8$ ont la signification déjà indiquée, que l'on isole et, si désiré, salifie.

11. Procédé selon la revendication 10, caractérisé en ce que :

a) la réaction du produit de formule (A) avec le produit de formule (XI) ou avec le réactif de formule $R_7$—Li est effectuée au sein de l'éther anhydre,

b) la déshydratation du produit de formule ($I_D$) est effectuée au moyen d'acide chlorhydrique concentré.

12. Procédé selon la revendication 1, pour la préparation des produits de formule (I) dans laquelle $R_1$, $R_3$ et $R_5$ sont définis comme précédemment, $R_2$ représente un radical benzoyle ou un groupement : $—C{=}CH—R_8$, $R_8$ étant défini comme précédemment et $R_4$ représente un radical hydroxy ou un radical acyloxy renfermant de 2 à 8 atomes de carbone, ainsi que de leurs sels, caractérisé en ce que l'on hydrolyse un produit correspondant dans lequel $R_4$ représente un radical alcoxy renfermant de 1 à 8 atomes de carbone, pour obtenir un produit de formule ($I_G$) :

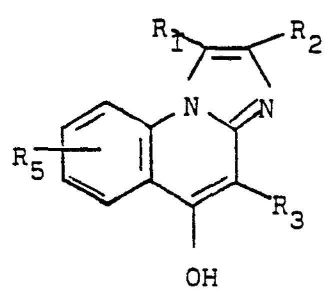

($I_G$)

dans laquelle $R_1$, $R_2$, $R_3$ et $R_5$ ont la signification déjà indiquée et que,

soit on isole et, si désiré, salifie le produit de formule ($I_G$) ainsi obtenu,

soit on fait réagir ce dernier avec un halogénure de formule (XII) :

Hal—$R_9$ (XII)

dans laquelle $R_9$ représente un radical acyle renfermant de 2 à 8 atomes de carbone et Hal représente un atome de chlore ou de brome, ou avec un anhydride d'acide de formule (XIII) :

$(R_9)_2O$ (XIII)

dans laquelle $R_9$ a la signification déjà indiquée, pour obtenir un produit de formule ($I_H$) :

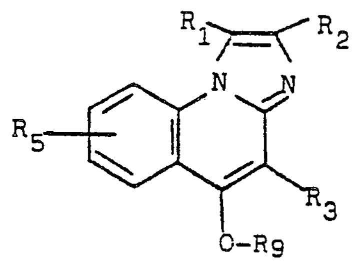

($I_H$)

dans laquelle $R_1$, $R_2$, $R_3$, $R_9$ et $R_5$ ont la signification déjà indiquée, que l'on isole et, si désiré, salifie.

13. Procédé selon la revendication 12, caractérisé en ce que :

a) l'hydrolyse du produit dans lequel $R_4$ représente un radical alcoxy est effectuée au moyen de l'acide bromhydrique par chauffage au reflux du milieu réactionnel pendant trente heures en présence d'eau ;

b) la réaction du produit de formule ($I_G$) avec l'halogénure de formule (XII) ou l'anhydride d'acide de formule (XIII) est effectuée au sein de la triéthylamine.

14. Procédé selon l'une quelconque des revendications 1 à 13, caractérisé en ce que l'on utilise au départ un produit dans lequel $R_3$ représente un atome d'hydrogène ou un radical méthyle ou éthyle.

15. Procédé selon l'une quelconque des revendications 1 à 14, caractérisé en ce que l'on utilise au départ un produit dans lequel $R_5$ représente un radical éthyle et $R_3$ représente un radical méthyle ou éthyle.

16. Procédé selon la revendication 1, caractérisé en ce que l'on prépare l'un quelconque des produits de formule I dont les noms suivent :

la (7-éthyl-5-méthoxy-4-méthyl [imidazo] 1,2-a [quinoléin-2-yl]) phényl méthanone et ses sels,

la (4,7-diéthyl-5-méthoxy [imidazo] 1,2-a [quinoléin-2-yl]) phényl méthanone et ses sels.

**Claims** (for the Contracting States : BE, CH, DE, FR, IT, LI, LU, NL, SE)

1. Imidazo [1,2-a] quinolines and their salts, characterized in that they answer to the general formula (I) :

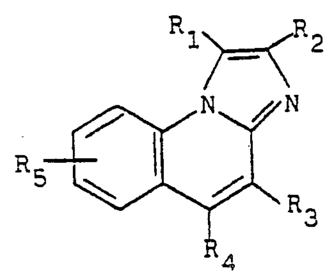

(I)

in which $R_1$ represents a hydrogen atom or a chlorine or bromine atom, $R_2$ represents a benzoyl radical, a

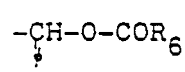

radical in which $R_6$ represents an alkyl radical containing from 1 to 5 carbon atoms, a radical :

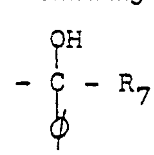

in which $R_7$ represents an alkyl radical containing from 1 to 5 carbon atoms, or a

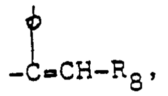

radical, in which $R_8$ represents a hydrogen atom or an alkyl radical containing from 1 to 4 carbon atoms, the symbol $\phi$ representing a phenyl radical, $R_3$ represents a hydrogen atom or an alkyl radical containing from 1 to 5 carbon atoms, $R_4$ represents an alkoxy radical containing from 1 to 8 carbon atoms, a hydroxy radical, an acyloxy radical containing from 2 to 8 carbon atoms, an alkyl radical containing from 1 to 8 carbon atoms, $R_5$ represents an alkyl radical containing from 1 to 8 carbon atoms, it being understood that $R_1$ and $R_3$ cannot represent a hydrogen atom when $R_2$ represents a benzoyl radical and $R_4$ represents an alkoxy radical containing from 1 to 8 carbon atoms.

2. Derivatives answering to the formula (I) of claim 1 as well as their salts, characterized in that in the said formula (I), $R_3$ represents a hydrogen atom or a methyl or ethyl radical, $R_4$ represents a methoxy, hydroxy, acetoxy or methyl radical, $R_5$ represents a methyl or ethyl radical, $R_1$ and $R_2$ having the significance already indicated.

3. Derivatives answering to the formula (I) of claim 1 as well as their salts, characterized in that in the said formula (I), $R_1$ represents a hydrogen atom, $R_2$ represents a benzoyl radical, a 1-phenyl vinyl radical, $R_3$ represents a hydrogen atom, a methyl or ethyl radical, $R_4$ represents a methoxy, hydroxy, acetoxy or methyl radical and $R_5$ represents an ethyl radical.

4. Derivatives answering to the formula (I) of claim 1, as well as their salts, characterized in that in the said formula (I), $R_1$ represents a hydrogen atom, $R_2$ represents a benzoyl radical, $R_3$ represents a methyl or ethyl radical, $R_4$ represents a methoxy radical, $R_5$ represents an ethyl radical.

5. (7-ethyl-5-methoxy-4-methyl [imidazo [1,2-a] quinoline-2-yl]) phenyl methanone and its salts.

6. (4,7-diethyl-5-methoxy [imidazo [1,2-a] quinoline-2-yl]) phenyl methanone and its salts.

7. Preparation process for products with the formula (I) in which $R_1$ represents a hydrogen atom, $R_2$ represents a benzoyl radical, $R_4$ represents an $R'_4$ radical which represents an alkoxy radical containing from 1 to 8 carbon atoms or an alkyl radical containing from 1 to 8 carbon atoms, $R_3$ and $R_5$ have the significance already indicated, as well as their salts, characterized in that a product with the formula (II) :

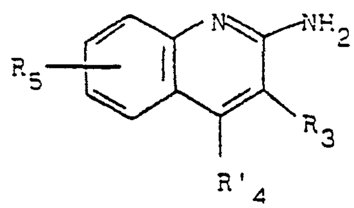

(II)

in which $R_3$, $R'_4$ and $R_5$ have the significance already indicated, is made to react with a product with the formula (III) :

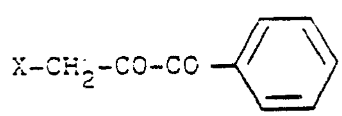

(III)

in which X represents a halogen atom, so as to obtain a product with the formula (IV) :

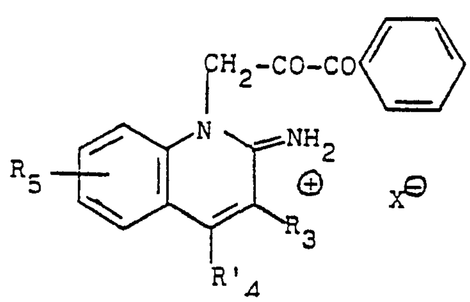

(IV)

in which $R_3$, $R'_4$ and $R_5$ have the significance already indicated, then the product with the formula (IV) obtained is cyclized so as to form the product with the formula ($I_A$) :

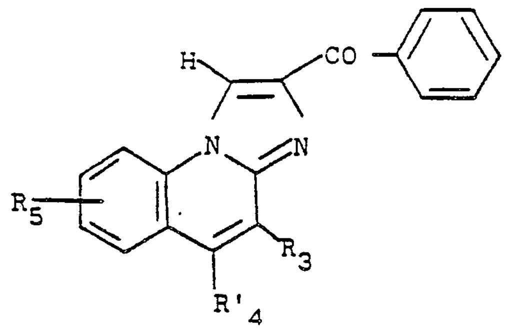

(I$_A$)

in which $R_3$, $R'_4$ and $R_5$ have the previously given significance, which is salified, if necessary or desired.

8. Process according to claim 7, characterized in that :

a) the reaction of the product with the formula (II) with the product with the formula (III) is carried out in an organic solvent such as dimethoxymethane ;

b) the cyclization of the product with the formula (IV) is carried out by heating in an organic solvent such as ethanol ;

c) the reaction of the product with the formula (II) with the product with the formula (III) is followed up to the product with the formula (I$_A$) without isolation of the product with the formula (IV).

9. Preparation process for products with the formula (I) in which $R_1$ represents a chlorine or bromine atom, $R_2$ represents a benzoyl radical, as well as for their salts, characterized in that a corresponding product in which $R_1$ represents a hydrogen atom is halogenated, so as to obtain a product with the formula (I$_B$) :

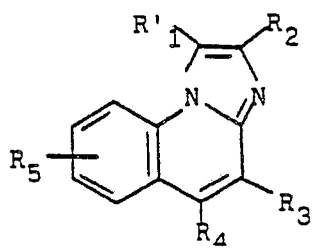

(I$_B$)

in which $R'_1$ represents a chlorine or bromine atom, $R_2$, $R_3$, $R_4$ and $R_5$ having the significance already indicated, which is isolated, and if desired, salified.

10. Process according to claim 9, characterized in that :

a) halogenation is carried out by means of N-bromosuccinimide when it is desired to prepare a product with the formula (I) in which $R_1$ represents a bromine atom,

b) halogenation is carried out by means of N-chlorosuccinimide when it is desired to prepare a product with the formula (I) in which $R_1$ represents a chlorine atom.

11. Preparation process for products with the formula (I) in which $R_4$ represents an $R'_4$ radical, $R_1$, $R_3$, $R'_4$ and $R_5$ have the significance already indicated, and $R_2$ represents a benzoyl radical, as well as for their salts, characterized in that a product with the formula (V) :

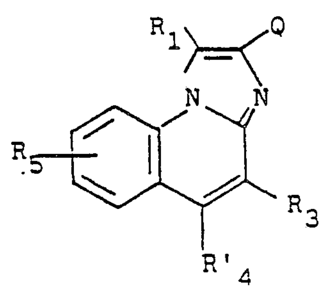

(V)

in which $R_1$, $R_3$, $R'_4$ and $R_5$ have the significance already indicated and Q represents an alkoxycarbonyl group containing from 2 to 4 carbon atoms or an aralkoxycarbonyl group containing from 8 to 12 carbon atoms, is reduced so as to obtain a product with the formula (VI) :

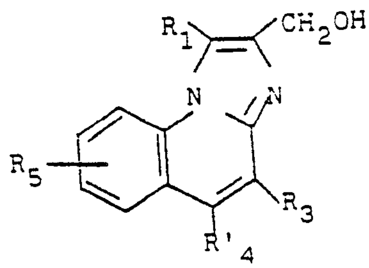

(VI)

in which $R_1$, $R_3$, $R'_4$ and $R_5$ have the significance already indicated, which is oxidized so as to obtain a product with the formula (VII) :

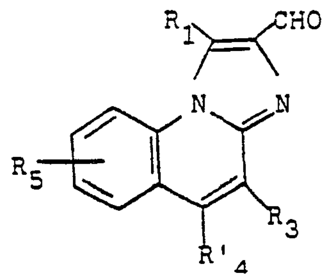

(VII)

in which $R_1$, $R_3$, $R'_4$ and $R_5$ have the significance already indicated, which is submitted to the action of a Grignard reagent formed starting from a product with the formula (VIII) :

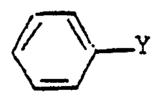

(VIII)

in which Y represents a chlorine, iodine or bromine atom, or to the action of phenyllithium, so as to obtain a product with the formula (IX) :

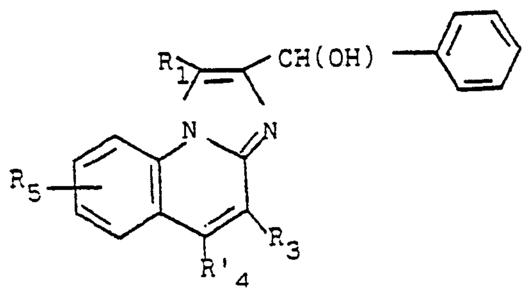

(IX)

in which $R_1$, $R_3$, $R'_4$ and $R_5$ have the significance already indicated, which is oxidized so as to obtain a product with the formula ($I_C$) :

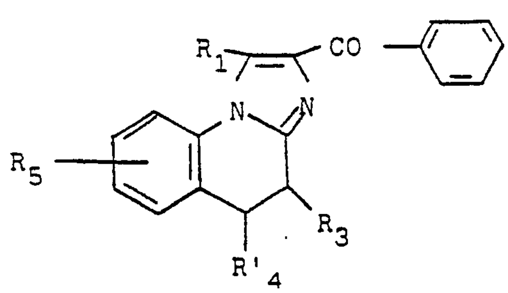

($I_C$)

in which $R_1$, $R_3$, $R'_4$ and $R_5$ have the significance already indicated, which is isolated and, if desired, salified.

12. Process according to claim 11, characterized in that :

a) the reduction of the product with the formula (V) is carried out by lithium-aluminium hydride, a mixture of sodium borohydride-aluminium chloride or lithium borohydride ;

b) the oxidation of the product with the formula (VI) is carried out by manganese dioxide or nitric acid, ferric chloride, a chromium oxide-pyridine mixture or by the Oppenauer method or by dehydrogenation on a catalyst with a copper base ;

c) the reaction of the product with the formula (VII) with the Grignard reagent or with phenyllithium is carried out in tetrahydrofuran ;

d) the oxidation of the product with the formula (IX) is carried out by one of the methods indicated above.

13. Preparation process for products with the formula (I) in which $R_2$ represents a radical :

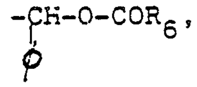

$R_1$, $R_3$, $R_5$ being defined as previously and $R_4$ being different from hydroxy, as well as for their salts, characterized in that a product with the formula :

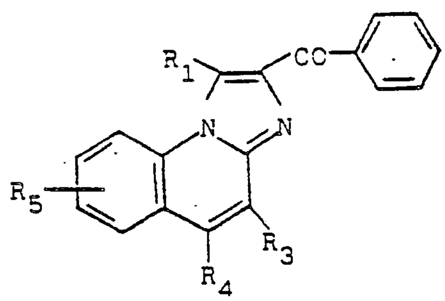

(A)

in which $R_1$, $R_3$, $R_4$ and $R_5$ are defined as previously, is reduced, so as to obtain a product with the formula (X) :

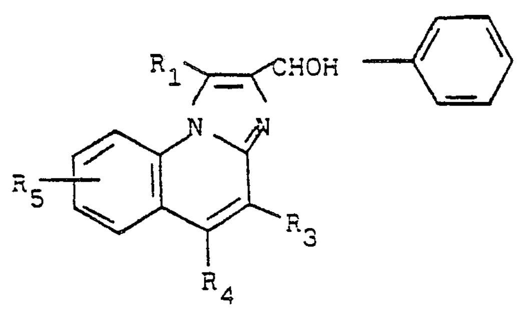

(X)

in which $R_1$, $R_3$, $R_4$ and $R_5$ are defined as previously, which is submitted to an acylation reaction, so as to obtain a product with the formula ($I_D$) :

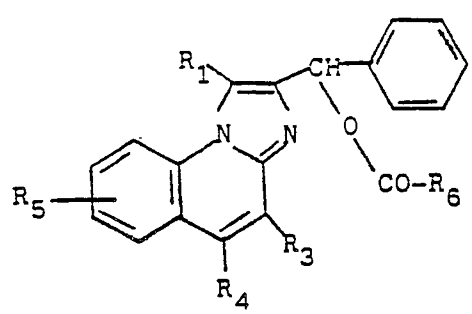

($I_D$)

in which $R_1$, $R_3$, $R_4$ and $R_5$ have the significance already indicated, which is isolated and, if desired, salified.

14. Process according to claim 13, characterized in that :

a) the reduction of the product with the formula (A) is carried out by means of sodium borohydride in an organic solvent such as ethanol,

b) the acylation of the product with the formula (X) is carried out in an acyl halogenide, in particular a chloride or a bromide, in pyridine.

15. Preparation process for products with the formula (I) in which $R_2$ represents a radical :

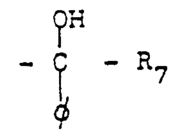

or a radical :

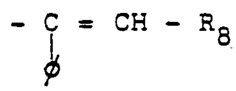

$R_1$, $R_3$, $R_5$, $R_7$ and $R_8$ being defined as previously and $R_4$ is different from acyloxy, as well as for their salts, characterized in that a product with the formula (A) as defined previously, is made to react with a Grignard reagent formed starting from a product with the formula (XI) :

$$Y—R_7 \qquad (XI)$$

or with a reagent with the formula $R_7$—Li, in which Y and $R_7$ are defined as previously, so as to obtain a product with the formula ($I_E$) :

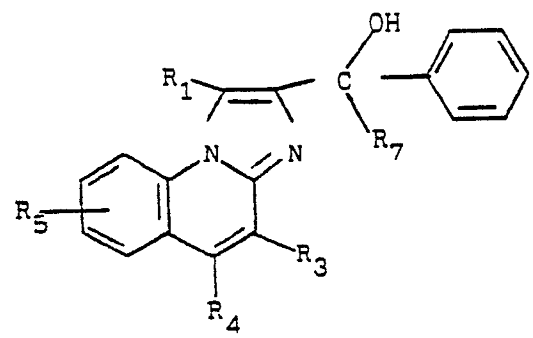

($I_E$)

in which $R_1$, $R_3$, $R_4$, $R_5$ and $R_7$ have the significance already indicated and in that

either the product with the formula ($I_E$) thus obtained is isolated and, if desired, salified,

or the latter is dehydrated in the case where $R_7$ represents a $—CH_2R_8$ radical, $R_8$ being defined as previously, so as to obtain a product with the formula ($I_F$) :

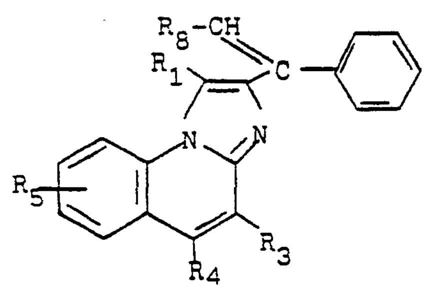

($I_F$)

in which $R_1$, $R_3$, $R_4$, $R_5$ and $R_8$ have the significance already indicated, which is isolated and, if desired, salified.

16. Process according to claim 15, characterized in that :

a) the reaction of the product with the formula (A) with the product with the formula (XI) or with the reagent with the formula $R_7$—Li is carried out in anhydrous ether,

b) the dehydration of the product with the formula ($I_D$) is carried out by means of concentrated hydrochloric acid.

17. Preparation process for products with the formula (I) in which $R_1$, $R_3$ and $R_5$ are defined as previously, $R_2$ represents a benzoyl radical or a

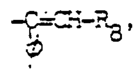

group, $R_8$ being defined as previously and $R_4$ represents a hydroxy radical or an acyloxy radical containing from 2 to 8 carbon atoms, as well as for their salts, characterized in that a corresponding product in which $R_4$ represents an alkoxy radical containing from 1 to 8 carbon atoms in hydrolyzed, so as to obtain a product with the formula ($I_G$) :

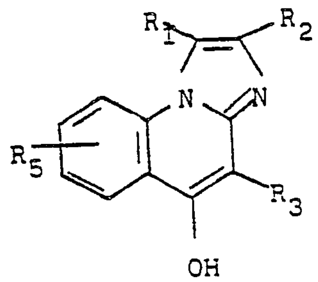

($I_G$)

in which $R_1$, $R_2$, $R_3$ and $R_5$ have the significance already indicated and in that

either the product with the formula ($I_G$) thus obtained is isolated and, if desired, salified,

or the latter is made to react with a halogenide with the formula (XII) :

$$Hal—R_9 \qquad (XII)$$

in which $R_9$ represents an acyl radical containing from 2 to 8 carbon atoms and Hal represents a chlorine or bromine atom, or with an acid anhydride with the formula (XIII) :

$$(R_9)_2O \qquad (XIII)$$

in which $R_9$ has the significance already indicated, so as to obtain a product with the formula ($I_H$) :

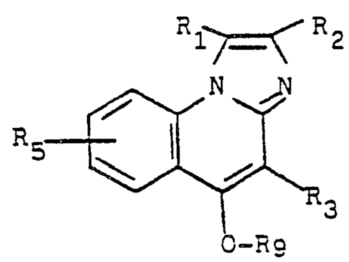

($I_H$)

in which $R_1$, $R_2$, $R_3$, $R_9$ and $R_5$ have the significance already indicated, which is isolated and, if desired, salified.

18. Process according to claim 17, characterized in that :

a) the hydrolysis of the product in which $R_4$ represents an alkoxy radical is carried out by means of hydrobromic acid by heating the reactional medium to reflux for 30 hours in the presence of water ;

b) the reaction of the product with the formula ($I_G$) with the halogenide with the formula (XII) or the anhydride acid with the formula (XIII) is carried out in triethylamine.

19. Medicaments, characterized in that they are constituted by new imidazo [1,2-a] quinolines as defined in claim 1 as well as by their salts of addition with pharmaceutically acceptable acids.

20. Medicaments, characterized in that they are constituted by new imidazo [1,2-a] quinolines as defined in claim 2, 3 or 4, as well as by their salts of addition with pharmaceutically acceptable acids.

21. Medicaments, characterized in that they are constituted by derivatives as defined in claim 5 or 6, as well as by the salts of addition with pharmaceutically acceptable acids of the said derivatives.

22. Pharmaceutical compositions, characterized in that they contain as active principle, at least one of the medicaments as defined in any one of the claims 19, 20 or 21.

**Claims** (for the Contracting State AT)

1. Preparation process for new imidazo [1,2-a] quinolines and for their salts, answering to the general formula (I) :

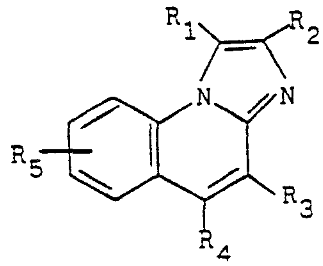

(I)

in which $R_1$ represents a hydrogen atom or a chlorine or bromine atom, $R_2$ represents a benzoyl radical, a

$-CH-O-COR_6$

radical in which $R_6$ represents an alkyl radical containing from 1 to 5 carbon atoms, a radical :

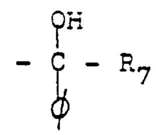

in which $R_7$ represents an alkyl radical containing from 1 to 5 carbon atoms, or a

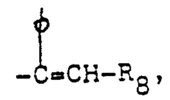

radical, in which $R_8$ represents a hydrogen atom or an alkyl radical containing from 1 to 4 carbon atoms, the symbol ϕ representing a phenyl radical, $R_3$ represents a hydrogen atom or an alkyl radical containing from 1 to 5 carbon atoms, $R_4$ represents an alkoxy radical containing from 1 to 8 carbon atoms, a hydroxy radical, an acyloxy radical containing from 2 to 8 carbon atoms, an alkyl radical containing from 1 to 8 carbon atoms, $R_5$ represents an alkyl radical containing from 1 to 8 carbon atoms, it being understood that $R_1$ and $R_3$ cannot represent a hydrogen atom when $R_2$ represents a benzoyl radical and $R_4$ represents an alkoxy radical containing from 1 to 8 carbon atoms, characterized in that :

for the preparation of products with the formula (I) in which $R_1$ represents a hydrogen atom, $R_2$ represents a benzoyl radical, $R_4$ represents an $R'_4$ radical, which represents an alkoxy radical containing from 1 to 8 carbon atoms or an alkyl radical containing from 1 to 8 carbon atoms, $R_3$ and $R_5$ have the significance already indicated, as well as their salts, a product withf the formula (II) :

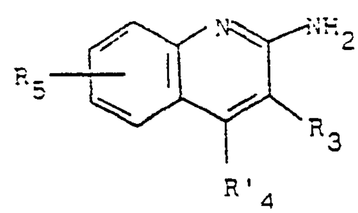

(II)

in which $R_3$, $R'_4$ and $R_5$ have the significance already indicated, is made to react with a product with the formula (III) :

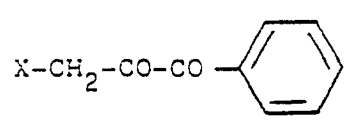

(III)

in which X represents a halogen atom, so as to obtain a product with the formula (IV) :

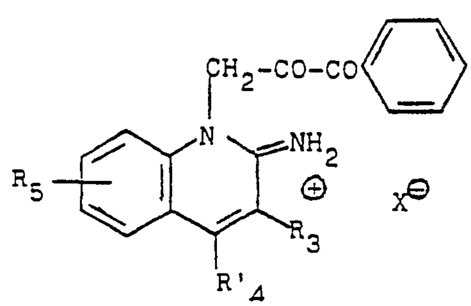

(IV)

in which $R_3$, $R'_4$ and $R_5$ have the significance already indicated, then the product with the formula (IV) obtained is cyclized in order to form the product with the formula ($I_A$) :

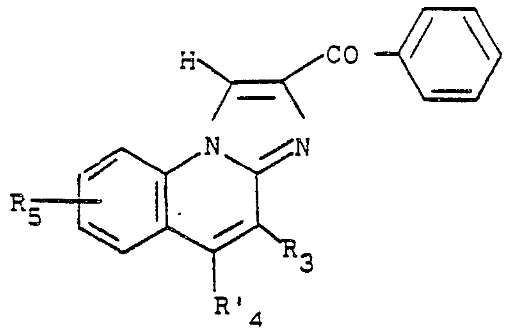

($I_A$)

in which $R_3$, $R'_4$ and $R_5$ have the previously given significance, which is salified, if necessary or desired ;

for the preparation of products with the formula (I) in which $R_1$ represents a chlorine or bromine atom, $R_2$ represents a benzoyl radical, as well as of their salts, a corresponding product in which $R_1$ represents a hydrogen atom is halogenated, so as to obtain a product with the formula ($I_B$) :

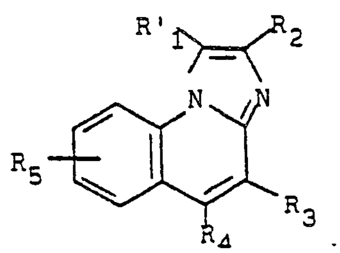

($I_B$)

in which $R'_1$ represents a chlorine or bromine atom, $R_2$, $R_3$, $R_4$ and $R_5$ having the significance already indicated, which is isolated, and, if desired, salified.

for the preparation of products with the formula (I) in which $R_4$ represents an $R'_4$ radical, $R_1$, $R_3$, $R'_4$ and $R_5$ have the significance already indicated, and $R_2$ represents a benzoyl radical, as well as of their salts, a product with the formula (V) :

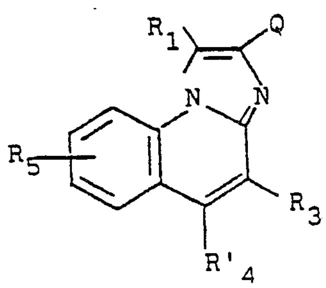

(V)

in which $R_1$, $R_3$, $R'_4$ and $R_5$ have the significance already indicated and Q represents an alkoxycarbonyl group containing from 2 to 4 carbon atoms or an aralkoxycarbonyl group containing from 8 to 12 carbon atoms, is reduced, so as to obtain a product with the formula (VI) :

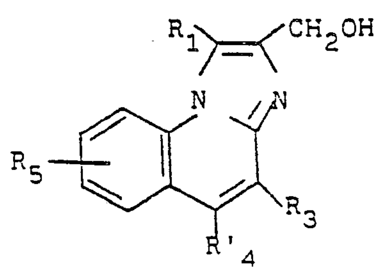

(VI)

in which $R_1$, $R_3$, $R'_4$ and $R_5$ have the significance already indicated, which is oxidized so as to obtain a product with the formula (VII) :

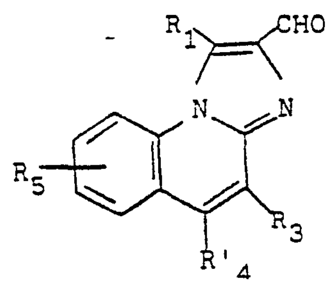

(VII)

in which $R_1$, $R_3$, $R'_4$ and $R_5$ have the significance already indicated, which is submitted to the action of a Grignard reagent formed starting from a product with the formula (VIII) :

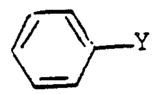

(VIII)

in which Y represents a chlorine, iodine or bromine atom, or to the action of phenyllithium, so as to obtain a product with the formula (IX) :

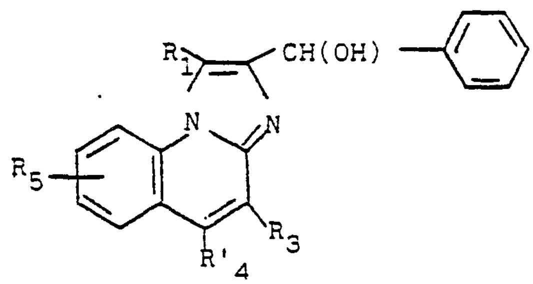

(IX)

in which $R_1$, $R_3$, $R'_4$ and $R_5$ have the significance already indicated, which is oxidized so as to obtain a product with the formula ($I_C$) :

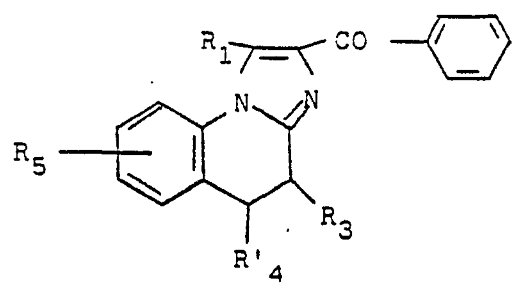

($I_C$)

in which $R_1$, $R_3$, $R'_4$ and $R_5$ have the significance already indicated, which is isolated and, if desired, salified,

for the preparation of products with the formula (I) in which $R_2$ represents a

$$-\underset{\phi}{CH}-O-COR_6,$$

radical, $R_1$, $R_3$ and $R_5$ being defined as previously and $R_4$ being different from hydroxy, as well as of their salts, a product with the formula :

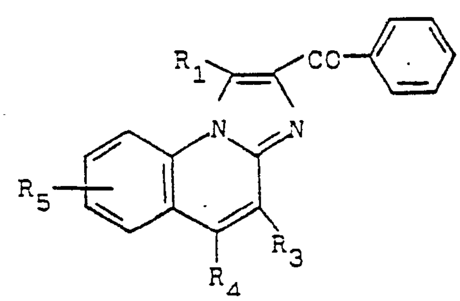

(A)

in which $R_1$, $R_3$, $R_4$ and $R_5$ are defined as previously, is reduced, so as to obtain a product with the formula (X) :

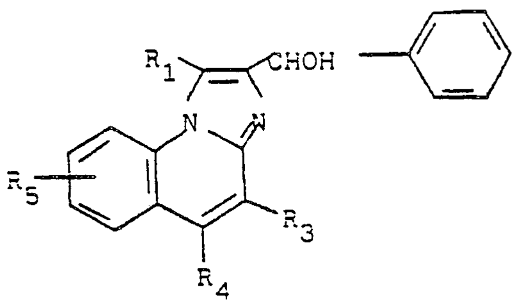

(X)

in which $R_1$, $R_3$, $R_4$ and $R_5$ are defined as previously, which is submitted to an acylation reaction, so as to obtain a product with the formula ($I_D$) :

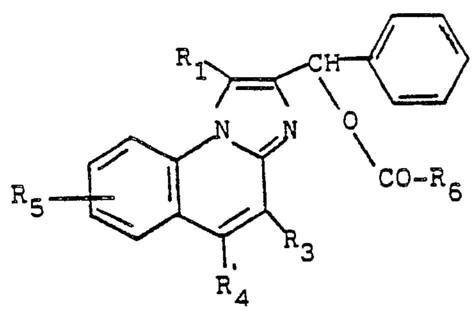

($I_D$)

in which $R_1$, $R_3$, $R_4$ and $R_5$ have the significance already indicated, which is isolated and, if desired, salified ;

for the preparation of products with the formula (I) in which $R_2$ represents a radical :

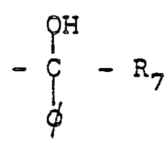

or a radical :

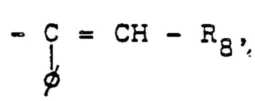

$R_1$, $R_3$, $R_5$, $R_7$ and $R_8$ being defined as previously and $R_4$ is different from acyloxy, as well as of their salts, a product with the formula (A) as defined previously is made to react with a Grignard reagent formed starting from a product with the formula (XI) :

$$Y—R_7 \qquad (XI)$$

or with a reagent with the formula : $R_7$—Li in which Y and $R_7$ are defined as previously, so as to obtain a oroduct with the formula ($I_E$) :

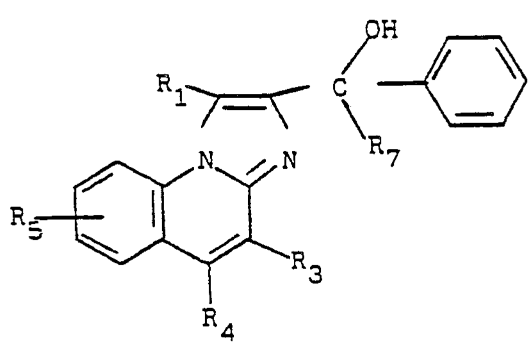

($I_E$)

in which $R_1$, $R_3$, $R_4$, $R_5$ and $R_7$ have the significance already indicated and

either the product with the formula ($I_E$) thus obtained is isolated and, if desired, salified,

or the latter is dehydrated in the case where $R_7$ represents a $—CH_2R_8$ radical, $R_8$ being defined as previously, so as to obtain a product with the formula ($I_F$) :

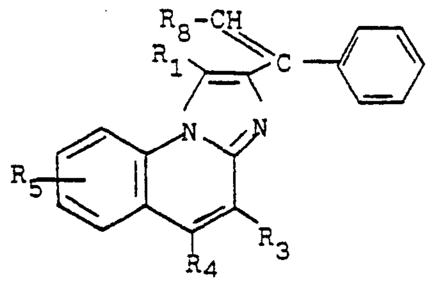

($I_F$)

in which $R_1$, $R_3$, $R_4$, $R_5$ and $R_8$ have the significance already indicated, which is isolated and, if desired, salified ;

for the preparation of products with the formula (I) in which $R_1$, $R_3$ and $R_5$ are defined as previously, $R_2$ represents a benzoyl radical or a group :

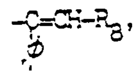

$R_8$ being defined as previously and $R_4$ represents a hydroxy radical or an acyloxy radical containing from 2 to 8 carbon atoms, as well as of their salts, a corresponding product in which $R_4$ represents an alkoxy radical containing from 1 to 8 carbon atoms is hydrolized, so as to obtain a product with the formula ($I_G$) :

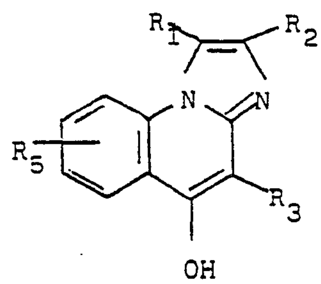

($I_G$)

in which $R_1$, $R_2$, $R_3$ and $R_5$ have the significance already indicated and

either the product with the formula ($I_G$) thus obtained is isolated and, if desired, salified,

or the latter is made to react with a halogenide with the formula (XII) :

$$Hal—R_9 \qquad (XII)$$

in which $R_9$ represents an acyl radical containing from 2 to 8 carbon atoms and Hal represents a chlorine or bromine atom, or with an acid anhydride with the formula (XIII) :

$$(R_9)_2O \qquad (XIII)$$

in which $R_9$ has the significance already indicated, so as to obtain a product with the formula ($I_H$) :

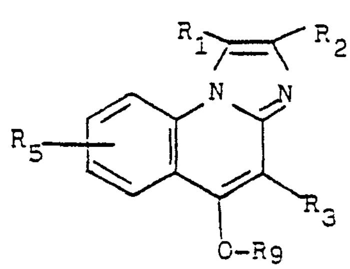

($I_H$)

in which $R_1$, $R_2$, $R_3$, $R_9$ and $R_5$ have the significance already indicated which is isolated and, if desired, salified.

2. Process according to claim 1, for the preparation of products with the formula (I) in which $R_1$ represents a hydrogen atom, $R_2$ represents a benzoyl radical, $R_4$ represents an $R'_4$ radical which represents an alkoxy radical containing from 1 to 8 carbon atoms or an alkyl radical containing from 1 to 8 carbon atoms, and $R_3$ and $R_5$ have the significance already indicated, as well as of their salts, characterized in that a product with the formula (II) :

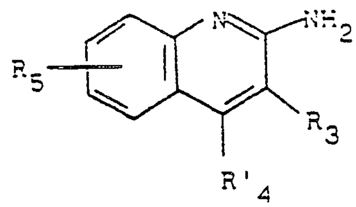

(II)

in which $R_3$, $R'_4$ and $R_5$ have the significance already indicated, is made to react with a product with the formula (III) :

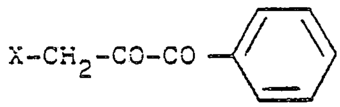

(III)

in which X represents a halogen atom, so as to obtain a product with the formula (IV) :

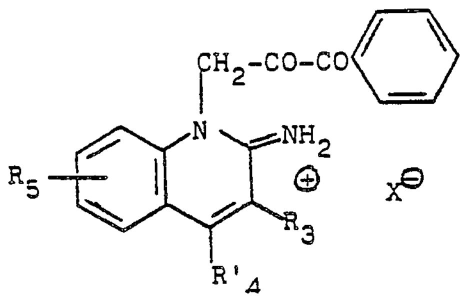

(IV)

in which $R_3$, $R'_4$ and $R_5$ have the significance already indicated, then the product with the formula (IV) obtained is cyclized in order to form the product with the formula ($I_A$) :

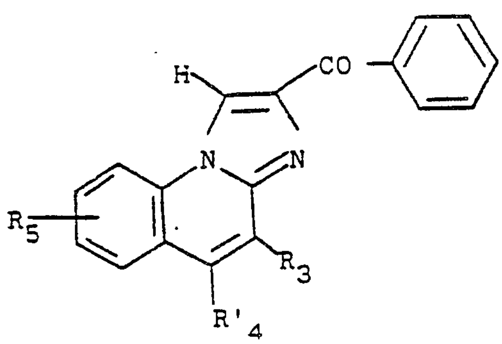

($I_A$)

in which $R_3$, $R'_4$ and $R_5$ have the previously given significance, which is salified, if necessary or desired.

3. Process according to claim 2, characterized in that :

a) the reaction of the product with the formula (II) with the product with the formula (III) is carried out in an organic solvent such as dimethoxymethane ;

b) the cyclization of the product with the formula (IV) is carried out by heating in an organic solvent such as ethanol ;

c) the reaction of the product with the formula (II) with the product with the formula (III) is followed up to the product with the formula ($I_A$) without isolation of the product with the formula (IV).

4. Process according to claim 1, for the preparation of products with the formula (I) in which $R_1$ represents a chlorine or bromine atom, $R_2$ represents a benzoyl radical, as well as of their salts, characterized in that a corresponding product in which $R_1$ represents a hydrogen atom is halogenated, so as to obtain a product with the formula ($I_B$) :

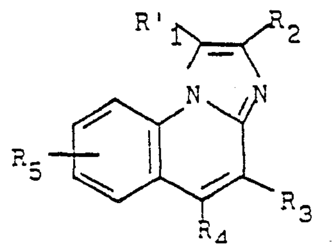

($I_B$)

in which $R'_1$ represents a chlorine or bromine atom, $R_2$, $R_3$, $R_4$ and $R_5$ having the significance already indicated, which is isolated and, if desired, salified.

5. Process according to claim 4, characterized in that :

a) the halogenation is carried out by means of N-bromosuccinimide when it is desired to prepare a product with the formula (I) in which $R_1$ represents a bromine atom,

b) the halogenation is carried out by means of N-chlorosuccinimide when it is desired to prepare a product with the formula (I) in which $R_1$ represents a chlorine atom.

6. Process according to claim 1, for the preparation of products with the formula (I) in which $R_4$ represents an $R'_4$ radical, $R_1$, $R_3$, $R'_4$ and $R_5$ have the significance already indicated and $R_2$ represents a benzoyl radical, as well as of their salts, characterized in that a product with the formula (V) :

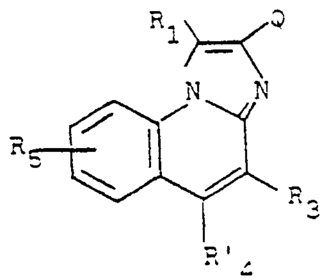

(V)

in which $R_1$, $R_3$, $R'_4$ and $R_5$ have the significance already indicated and Q represents an alkoxycarbonyl group containing from 2 to 4 carbon atoms or an aralkoxycarbonyl group containing from 8 to 12 carbon atoms, is reduced, so as to obtain a product with the formula (VI) :

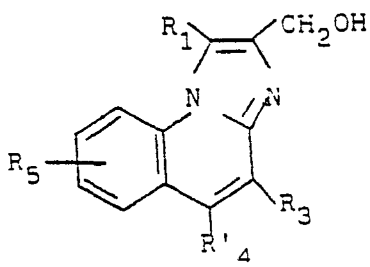

(VI)

in which $R_1$, $R_3$, $R'_4$ and $R_5$ have the significance already indicated, which is oxidized so as to obtain a product with the formula (VII) :

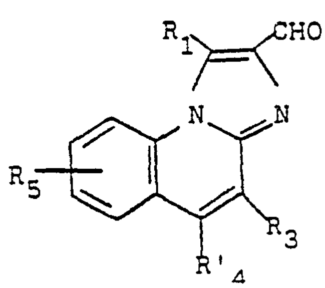

(VII)

in which $R_1$, $R_3$, $R'_4$ and $R_5$ have the significance already indicated, which is submitted to the action of a Grignard reagent formed starting from a product with the formula (VIII) :

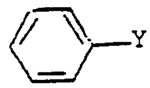

(VIII)

in which Y represents a chlorine, iodine or bromine atom, or to the action of phenyllithium, so as to obtain a product with the formula (IX) :

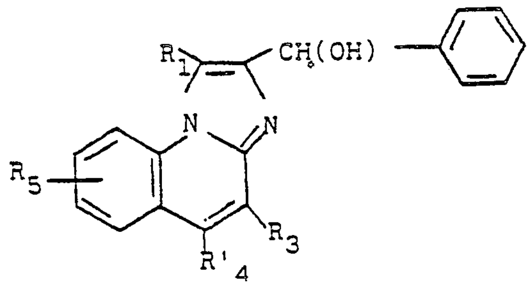

(IX)

in which $R_1$, $R_3$, $R'_4$ and $R_5$ have the significance already indicated, which is oxidized so as to obtain a product with the formula ($I_C$) :

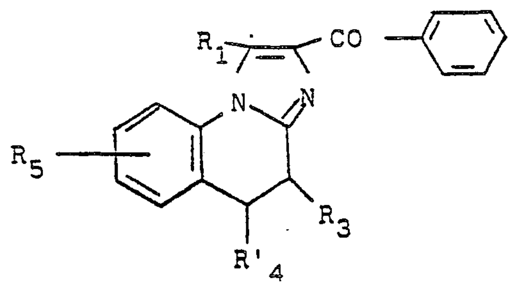

($I_C$)

in which $R_1$, $R_3$, $R'_4$ and $R_5$ have the significance already indicated, which is isolated and, if desired, salified.

7. Process according to claim 6, characterized in that :

a) the reduction of the product with the formula (V) is carried out by lithium-aluminium hybride, a mixture of sodium borohydride-aluminium chloride or lithium borohydride ;

b) the oxidation of the product with the formula (VI) is carried out by manganese dioxide or nitric acid, ferric chloride, a chromium oxide-pyridine mixture, or by the Oppenauer method or by dehydrogenation on a catalyst with a copper base ;

c) the reaction of the product with the formula (VII) with the Grignard reagent or with phenyllithium is carried out in tetrahydrofuran ;

d) the oxidation of the product with the formula (IX) is carried out by one of the methods indicated above.

8. Process according to claim 1, for the preparation of products with the formula (I) in which $R_2$ represents a radical :

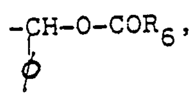

$R_1$, $R_3$, $R_5$ being defined as previously and $R_4$ being different from hydroxy, as well as of their salts, characterized in that a product with the formula :

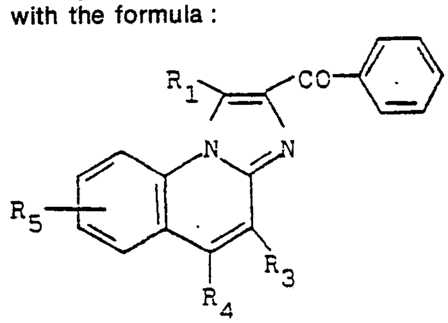

(A)

in which $R_1$, $R_3$, $R_4$ and $R_5$ are defined as previously, is reduced, so as to obtain a product with the formula (X) :

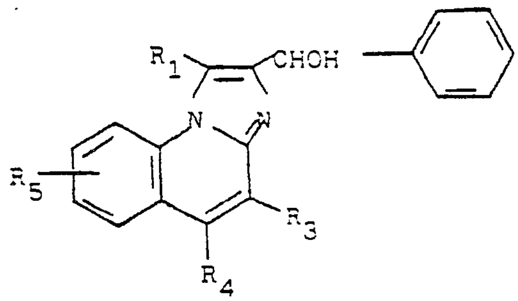

(X)

in which $R_1$, $R_3$, $R_4$ and $R_5$ are defined as previously, which is submitted to an acylation reaction, so as to obtain a product with the formula ($I_D$) :

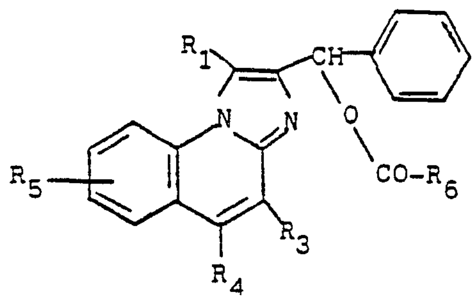

($I_D$)

in which $R_1$, $R_3$, $R_4$ and $R_5$ have the significance already indicated which is isolated and, if desired, salified.

9. Process according to claim 8, characterized in that :

a) the reduction of the product with the formula (A) is carried out by means of sodium borohydride in an organic solvent such as ethanol,

b) the acylation of the product with the formula (X) is caried out by means of an acyl halogenide, in particular a chloride or bromide in pyridine.

10. Process according to claim 1, for the preparation of products with the formula (I) in which $R_2$ represents a radical :

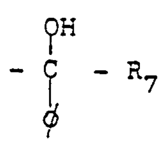

or a radical

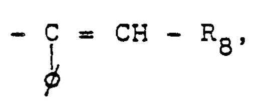

$R_1$, $R_3$, $R_5$, $R_7$ and $R_8$ being defined as previously and $R_4$ is different from acyloxy as well as of their salts, characterized in that a product with the formula (A) as defined previously is made to react with a Grignard reagent formed starting from a product with the formula (XI) :

$$Y—R_7 \qquad \text{(XI)}$$

or with a reagent with the formula : $R_7$—Li in which Y and $R_7$ are defined as previously, so as to obtain a product with the formula ($I_E$) :

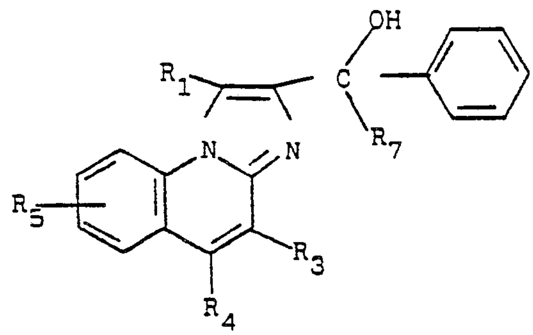

($I_E$)

in which $R_1$, $R_3$, $R_4$, $R_5$ and $R_7$ have the significance already indicated and

either the product with the formula ($I_E$) thus obtained is isolated and, if desired, salified,

or the latter is dehydrated in the case where $R_7$ represents a $—CH_2R_8$ radical, $R_8$ being defined as previously, so as to obtain a product with the formula ($I_F$) :

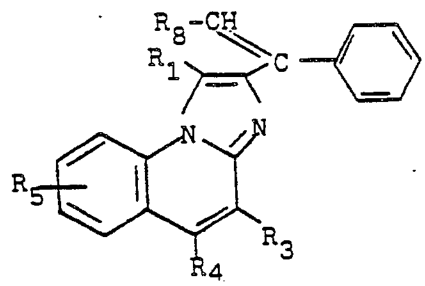

($I_F$)

in which $R_1$, $R_3$, $R_4$, $R_5$ and $R_8$ have the significance already indicated, which is isolated and, if desired, salified.

11. Process according to claim 10, characterized in that :

a) the reaction of the product with the formula (A) with the product with the formula (XI) or with the reagent with the formula $R_7—Li$ is carried out in anhydrous ether,

b) the dehydration of the product with the formula ($I_D$) is carried out by means of concentrated hydrochloric acid.

12. Process according to claim 1, for the preparation of products with the formula (I) in which $R_1$, $R_3$ and $R_5$ are defined as previously, $R_2$ represents a benzoyl radical or a group : $—C{=}CH—R_8$, $R_8$ being defined as previously and $R_4$ represents a hydroxy radical or an acyloxy radical containing from 2 to 8 carbon atoms, as well as of their salts, characterized in that a corresponding product in which $R_4$ represents an alkoxy radical containing from 1 to 8 carbon atoms is hydrolysed, so as to obtain a product with the formula ($I_G$) :

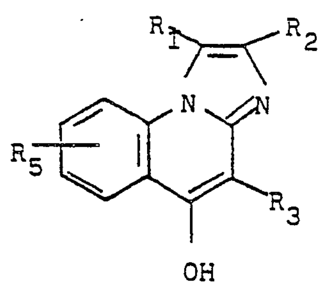

($I_G$)

in which $R_1$, $R_2$, $R_3$ and $R_5$ have the significance already indicated and

either the product with the formula ($I_G$) thus obtained is isolated and, if desired, salified,

or the latter is made to react with a halogenide with the formula (XII) :

$$Hal—R_9 \qquad (XII)$$

in which $R_9$ represents an acyl radical containing from 2 to 8 carbon atoms and Hal represents a chlorine or bromine atom, or with an acid anhydride with the formula (XIII):

$$(R_9)_2O \qquad \text{(XIII)}$$

in which $R_9$ has the significance already indicated, so as to obtain a product with the formula ($I_H$):

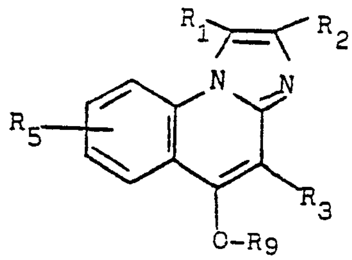

($I_H$)

in which $R_1$, $R_2$, $R_3$, $R_9$ and $R_5$ have the significance already indicated, which is isolated and, if desired, salified.

13. Process according to claim 12, characterized in that:

a) the hydrolysis of the product in which $R_4$ represents an alkoxy radical is carried out by means of hydrobromic acid by heating the reactional medium to reflux for thirty hours in the presence of water;

b) the reaction of the product with the formula ($I_G$) with the halogenide with the formula (XII) or the acid anhydride with the formula (XIII) is carried out in triethylamine.

14. Process according to any one of the claims 1 to 13, characterized in that at the start a product is used in which $R_3$ represents a hydrogen atom or a methyl or ethyl radical.

15. Process according to any one of the claims 1 to 14, characterized in that at the start a product is used in which $R_5$ represents an ethyl radical and $R_3$ represents a methyl or ethyl radical.

16. Process according to claim 1, characterized in that either one of the products with the formula (I) is prepared, of which the names follow:

(7-ethyl-5-methoxy-4-methyl [imidazo [1,2-a] quinoline-2-yl]) phenyl methanone and its salts,

(4,7-diethyl-5-methoxy [imidazo [1,2-a] quinoline-2-yl]) phenyl methanone and its salts.

**Patentansprüche** (für die Vertragsstaaten: BE, CH, DE, FR, IT, LI, LU, NL, SE)

1. Imidazo [1,2-a] chinoline und ihre Salze, dadurch gekennzeichnet, daß sie der allgemeinen Formel (I)

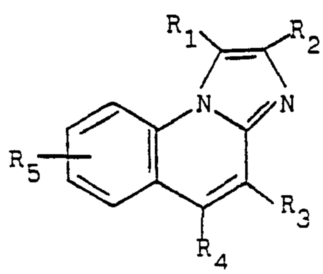

(I)

entsprechen, worin $R_1$ ein Wasserstoffatom oder ein Chlor- oder Bromatom bedeutet, $R_2$ einen Benzoylrest, einen Rest

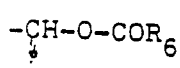

wobei $R_6$ einen Alkylrest mit 1 bis 5 Kohlenstoffatomen darstellt, einen Rest

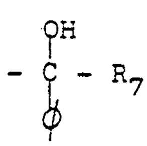

wobei $R_7$ einen Alkylrest mit 1 bis 5 Kohlenstoffatomen darstellt, oder einen Rest

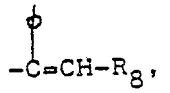

bedeutet, wobei $R_8$ ein Wasserstoffatom oder einen Alkylrest mit 1 bis 4 Kohlenstoffatomen darstellt, das Symbol ϕ einen Phenylrest bedeutet, $R_3$ ein Wasserstoffatom oder einen Alkylrest mit 1 bis 5 Kohlenstoffatomen bedeutet, $R_4$ einen Alkoxyrest mit 1 bis 8 Kohlenstoffatomen, einer Hydroxyrest, einen Acyloxyrest mit 2 bis 8 Kohlenstoffatomen, einen Alkylrest mit 1 bis 8 Kohlenstoffatomen bedeutet, $R_5$ einen Alkylrest mit 1 bis 8 Kohlenstoffatomen bedeutet, mit der Maßgabe, daß $R_1$ und $R_3$ nicht die Bedeutung eines Wasserstoffatoms haben können, wenn $R_2$ einen Benzoylrest darstellt und $R_4$ einen Alkoxyrest mit 1 bis 8 Kohlenstoffatomen bedeutet.

2. Derivate entsprechend der Formel (I) gemäß Anspruch 1 sowie ihre Salze, dadurch gekennzeichnet, daß in der genannten Formel (I) $R_3$ ein Wasserstoffatom oder einen Methyl- oder Ethylrest bedeutet, $R_4$ für eine Methoxy-, Hydroxy-, Acetoxy- oder Methylgruppe steht, $R_5$ einen Methyl- oder Ethylrest darstellt und $R_1$ und $R_2$ die angegebene Bedeutung besitzen.

3. Derivate entsprechend der Formel (I) gemäß Anspruch 1 sowie ihre Salze, dadurch gekennzeichnet, daß in der genannten Formel (I) $R_1$ für ein Wasserstoffatom steht, $R_2$ einen Benzoyl-, 1-Phenylvinylrest bedeutet, $R_3$ ein Wasserstoffatom, einen Methyl- oder Ethylrest darstellt, $R_4$ einen Methoxy-, Hydroxy-, Acetoxy- oder Methylrest bedeutet und $R_5$ für einen Ethylrest steht.

4. Derivate entsprechend der Formel (I) gemäß Anspruch 1 sowie ihre Salze, dadurch gekennzeichnet, daß in der genannten Formel (I) $R_1$ ein Wasserstoffatom bedeutet, $R_2$ für einen Benzoylrest steht, $R_3$ einen Methyl- oder Ethylrest bedeutet, $R_4$ einen Methoxyrest darstellt und $R_5$ für einen Ethylrest steht.

5. (7-Ethyl-5-methoxy-4-methyl [imidazo [1,2-a] chinolin-2-yl]) phenylmethanon und seine Salze.

6. (4,7-Diethyl-5-methoxy [imidazo [1,2-a] chinolin-2-yl]) phenylmethanon und seine Salze.

7. Verfahren zur Herstellung der Produkte der Formel (I), worin $R_1$ für ein Wasserstoffatom steht, $R_2$ einen Benzoylrest bedeutet, $R_4$ einen Rest $R'_4$ darstellt, welcher einen Alkoxyrest mit 1 bis 8 Kohlenstoffatomen oder einen Alkylrest mit 1 bis 8 Kohlenstoffatomen bedeutet, $R_3$ und $R_5$ die bereits angegebene Bedeutung haben, sowie ihrer Salze, dadurch gekennzeichnet, daß man ein Produkt der Formel (II)

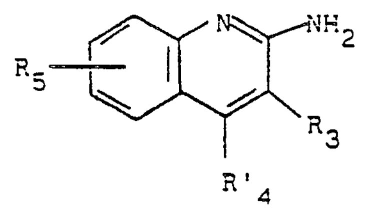

(II)

worin $R_3$, $R'_4$ und $R_5$ die angegebene Bedeutung haben, mit einem Produkt der Formel (III)

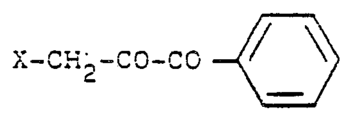

(III)

worin X ein Halogenatom darstellt, zur Reaktion bringt, um ein Produkt der Formel (IV)

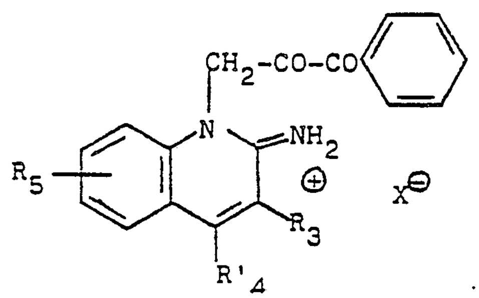

(IV)

zu erhalten, worin $R_3$, $R'_4$ und $R_5$ die angegebenen Bedeutungen besitzen, dann das erhaltene Produkt der Formel (IV) cyclisiert, um ein Produkt der Formel ($I_A$)

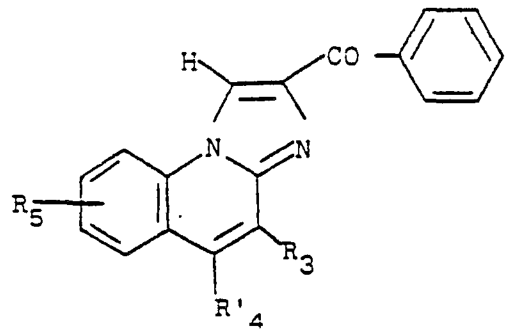

$(I_A)$

zu bilden, worin $R_3$, $R'_4$ und $R_5$ die angegebene Bedeutung haben, das man gegebenenfalls versalzt.

8. Verfahren gemäß Anspruch 7, dadurch gekennzeichnet, daß

a) die Reaktion des Produkts der Formel (II) mit dem Produkt der Formel (III) in einem organischen Lösungsmittel, wie Dimethoxymethan, durchgeführt wird ;

b) die Cyclisierung des Produkts der Formel (IV) durch Erhitzen in einem organischen Lösungsmittel, wie Ethanol, durchgeführt wird ;

c) die Reaktion des Produkts der Formel (II) mit dem Produkt der Formel (III) bis zum Produkt der Formel $(I_A)$ fortgesetzt wird, ohne das Produkt der Formel (IV) zu isolieren.

9. Verfahren zur Herstellung der Produkte der Formel (I), worin $R_1$ für ein Chlor- oder Bromatom steht, $R_2$ einen Benzoylrest bedeutet, sowie ihrer Salze, dadurch gekennzeichnet, daß man ein entsprechendes Produkt, in welchem $R_1$ für ein Wasserstoffatom steht, halogeniert, um ein Produkt der Formel $(I_B)$

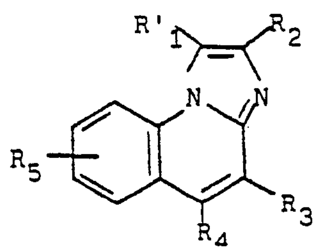

$(I_B)$

zu erhalten, worin $R'_1$ für ein Chlor- oder Bromatom steht und $R_2$, $R_3$, $R_4$ und $R_5$ die vorstehenden Bedeutungen besitzen, das man isoliert und gewünschtenfalls versalzt.

10. Verfahren gemäß Anspruch 9, dadurch gekennzeichnet, daß

a) die Halogenierung mit Hilfe von N-Bromsuccinimid durchgeführt wird, wenn man ein Produkt der Formel (I), worin $R_1$ ein Bromatom bedeutet, herstellen will ;

b) die Halogenierung mit Hilfe von N-Chlorsuccinimid durchgeführt wird, wenn man ein Produkt der Formel (I) erhalten will, worin $R_1$ für ein Chloratom steht.

11. Verfahren zur Herstellung der Produkte der Formel (I), worin $R_4$ für einen Rest $R'_4$ steht, $R_1$, $R_3$, $R'_4$ und $R_5$ die angegebene Bedeutung haben und $R_2$ einen Benzoylrest darstellt, sowie ihrer Salze, dadurch gekennzeichnet, daß man ein Produkt der Formel (V)

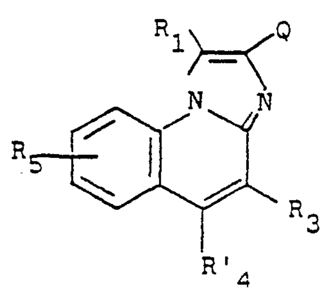

(V)

worin $R_1$, $R_3$, $R'_4$ und $R_5$ die angegebene Bedeutung haben und Q für eine Alkoxycarbonylgruppe mit 2

bis 4 Kohlenstoffatomen oder eine Aralkoxycarbonylgruppe mit 8 bis 12 Kohlenstoffatomen steht, reduziert, um ein Produkt der Formel (VI)

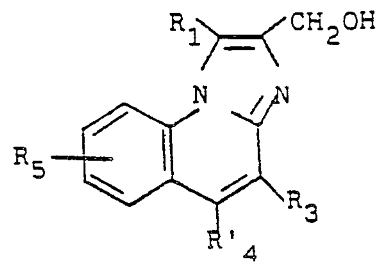

(VI)

zu erhalten, worin $R_1$, $R_3$, $R'_4$ und $R_5$ die angegebene Bedeutung besitzen, das man oxidiert, um ein Produkt der Formel (VII)

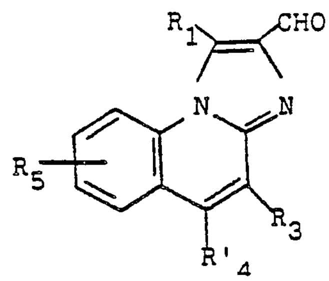

(VII)

zu erhalten, worin $R_1$, $R_3$, $R'_4$ und $R_5$ die obige Bedeutung besitzen, das man der Einwirkung eines Grignard-Reagens, gebildet aus einem Produkt der Formel (VIII)

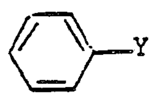

(VIII)

worin Y für ein Chlor-, Jod- oder Bromatom steht, oder der Einwirkung von Phenyllithium unterwirft, um ein Produkt der Formel (IX)

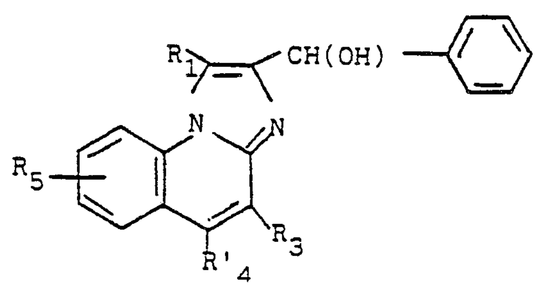

(IX)

zu erhalten, worin $R_1$, $R_3$, $R'_4$ und $R_5$ die angegebene Bedeutung haben, das man oxidiert, um ein Produkt der Formel ($I_C$)

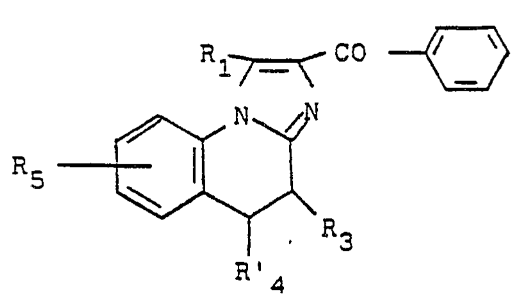

($I_C$)

zu erhalten, worin $R_1$, $R_3$, $R_4'$ und $R_5$ die angegebene Bedeutung besitzen, das man isoliert und gewünschtenfalls versalzt.

12. Verfahren gemäß Anspruch 11, dadurch gekennzeichnet, daß

a) die Reduktion des Produkts der Formel (V) durch Lithiumaluminiumhydrid, der Mischung aus Natriumborhydrid-Aluminiumchlorid oder Lithiumborhydrid bewirkt wird ;
b) die Oxidation des Produkts der Formel (VI) mit Hilfe von Mangandioxid durchgeführt wird oder auch mit Salpetersäure, Eisen (III)-chlorid, einer Chromoxid-Pyridin-Mischung oder auch durch das Oppenauer-Verfahren oder durch Dehydrogenierung über einem Katalysator auf Kupferbasis ;
c) die Reduktion des Produkts der Formel (VII) mit dem Grignard-Reagens oder mit Phenyllithium in Tetrahydrofuran durchgeführt wird ;
d) die Oxidation des Produkts der Formel (IX) durch eine der oben angegebenen Methoden bewirkt wird.

13. Verfahren zur Herstellung der Produkte der Formel (I), worin $R_2$ für einen Rest

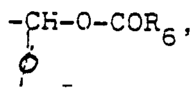

steht, $R_1$, $R_3$ und $R_5$ wie vorstehend definiert sind und $R_4$ von Hydroxy verschieden ist, sowie ihrer Salze, dadurch gekennzeichnet, daß man ein Produkt der Formel

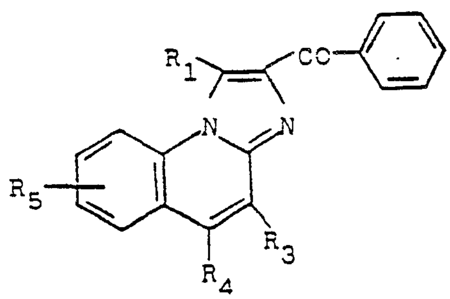

(A)

reduziert, worin $R_1$, $R_3$, $R_4$ und $R_5$ wie vorstehend definiert sind, um ein Produkt der Formel (X)

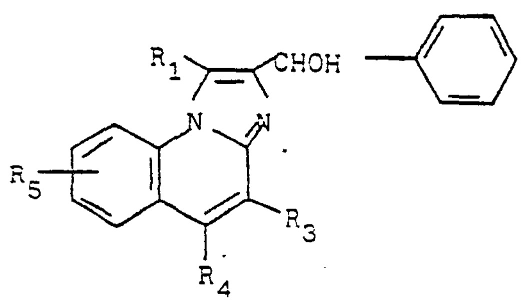

(X)

zu erhalten, worin $R_1$, $R_3$, $R_4$ und $R_5$ wie vorstehend definiert sind, das man einer Acylierungsreaktion unterwirft, um ein Produkt der Formel ($I_D$)

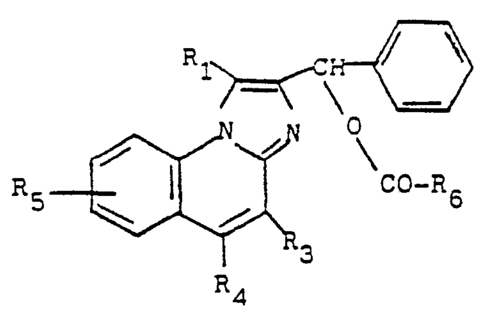

($I_D$)

zu erhalten, worin $R_1$, $R_3$, $R_4$ und $R_5$ die angegebenen Bedeutungen haben, das man isoliert und gewünschtenfalls versaltz.

14. Verfahren gemäß Anspruch 13, dadurch gekennzeichnet, daß

a) die Reduktion des Produkts der Formel (A) mit Hilfe von Natriumborhydrid in einem organischen

Lösungsmittel, wie Ethanol, durchgeführt wird,

b) die Acylierung des Produkts der Formel (X) mit Hilfe eines Acylhalogenids, insbesondere eines Chlorids oder eines Bromids, in Pyridin bewirkt wird.

15. Verfahren zur Herstellung der Produkte der Formel (I), worin $R_2$ für einen Rest

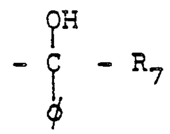

oder einen Rest

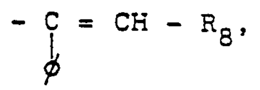

steht, $R_1$, $R_3$, $R_5$, $R_7$ und $R_8$ wie vorstehend definiert sind und $R_4$ von Acyloxy verschieden ist, und ihrer Salze, dadurch gekennzeichnet, daß man ein Produkt der Formel (A), wie vorstehend definiert, mit einem Grignard-Reagens, welches aus einem Produkt der Formel (XI) gebildet ist,

$$Y—R_7 \qquad \text{(XI)}$$

oder mit einem Reagens der Formel $R_7$—Li, worin Y und $R_7$ wie vorstehend definiert sind, zur Reaktion bringt, um ein Produkt der Formel $I_E$

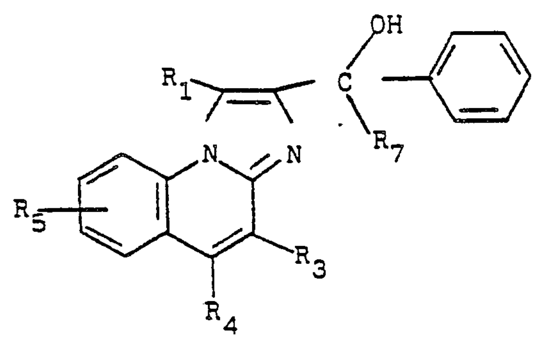

($I_E$)

zu erhalten, worin $R_1$, $R_3$, $R_4$, $R_5$ und $R_7$ die angegebenen Bedeutungen besitzen, und daß man entweder das so erhaltene Produkt der Formel ($I_E$) isoliert und gewünschtenfalls versalzt,

oder das letztere dehydratisiert, in dem Fall, daß $R_7$ einen Rest $—CH_2R_8$ bedeutet, wobei $R_8$ wie vorstehend definiert ist, um ein Produkt der Formel ($I_F$)

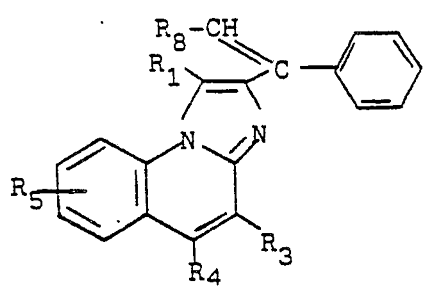

($I_F$)

zu erhalten, worin $R_1$, $R_3$, $R_4$, $R_5$ und $R_8$ die obigen Bedeutungen besitzen, welches man isoliert und gewünschtenfalls versalzt.

16. Verfahren gemäß Anspruch 15, dadurch gekennzeichnet, daß

a) die Reaktion des Produkts der Formel (A) mit dem Produkt der Formel (XI) oder mit dem Reagens der Formel $R_7$—Li in wasserfreiem Ether bewirkt wird,

b) die Dehydratisierung des Produkts der Formel ($I_D$) mit Hilfe von konzentrierter Chlorwasserstoffsäure bewirkt wird.

17. Verfahren zur Herstellung von Produkten der Formel (I), worin $R_1$, $R_3$ und $R_5$ die vorstehend angegebenen Bedeutungen haben, $R_2$ für einen Benzoylrest oder eine Gruppe

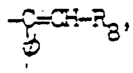

steht, wobei $R_8$ wie vorstehend definiert ist, und $R_4$ einen Hydroxyrest oder einen Acyloxyrest mit 2 bis 8 Kohlenstoffatomen bedeutet, sowie von deren Salzen, dadurch gekennzeichnet, daß man ein entsprechendes Produkt, in dem $R_4$ einen Alkoxyrest mit 1 bis 8 Kohlenstoffatomen darstellt, hydrolysiert, um ein Produkt der Formel ($I_G$)

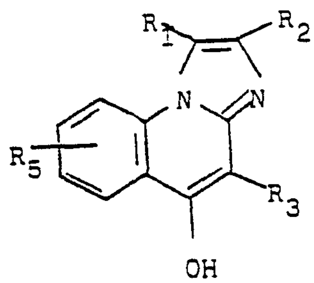

($I_G$)

worin $R_1$, $R_2$, $R_3$ und $R_5$ wie vorstehend definiert sind, zu erhalten, und daß man
entweder das so erhaltene Produkt der Formel ($I_G$) isoliert und gewünschtenfalls versalzt,
oder das letztere mit einem Halogenid der Formel (XII)

$$Hal—R_9 \qquad (XII)$$

worin $R_9$ einen Acylrest mit 2 bis 8 Kohlenstoffatomen bedeutet und Hal für ein Chlor- oder Bromatom steht, oder mit einem Säureanhydrid der Formel (XIII)

$$(R_9)_2O \qquad (XIII)$$

worin $R_9$ wie vorstehend definiert ist, zur Reaktion bringt, um ein Produkt der Formel ($I_H$)

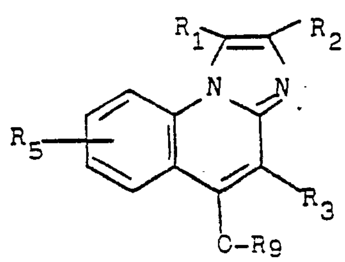

($I_H$)

zu erhalten, worin $R_1$, $R_2$, $R_3$, $R_9$ und $R_5$ die vorstehende Bedeutung haben, das man isoliert und gewünschtenfalls versalzt.

18. Verfahren gemäß Anspruch 17, dadurch gekennzeichnet, daß

a) die Hydrolyse des Produkts, worin $R_4$ einen Alkoxyrest bedeutet, mit Hilfe von Bromwasserstoffsäure durch Erhitzen des Reaktionsmilieus zum Rückfluß während 30 h in Gegenwart von Wasser bewirkt wird ;

b) die Reaktion des Produkts der Formel ($I_G$) mit dem Halogenid der Formel (XII) oder dem Säureanhydrid der Formel (XIII) in Triethylamin bewirkt wird.

19. Arzneimittel, dadurch gekennzeichnet, daß sie aus den neuen Imidazo [1,2-a] chinolinen, wie sie in Anspruch 1 definiert sind, zusammengesetzt sind sowie aus ihren Additionssalzen mit pharmazeutisch annehmbaren Säuren.

20. Arzneimittel, dadurch gekennzeichnet, daß sie aus den neuen Imidazo [1,2-a] chinolinen, wie sie in den Ansprüchen 2, 3 oder 4 definiert sind, zusammengesetzt sind sowie aus ihren Additionssalzen mit

pharmazeutisch annehmbaren Säuren.

21. Arzneimittel, dadurch gekennzeichnet, daß sie aus den Derivaten, wie in Anspruch 5 oder 6 definiert, zusammengesetzt sind sowie aus den Additionssalzen mit den pharmazeutisch annehmbaren Säuren der genannten Derivate.

22. Pharmazeutische Zusammensetzungen, dadurch gekennzeichnet, daß sie als Wirkstoff ein oder mehrere Arzneimittel gemäß einem der Ansprüche 19, 20 oder 21 enthalten.

**Patentansprüche** (für den Vertragsstaat AT)

1. Verfahren zur Herstellung neuer Imidazo [1,2-a] chinoline und ihrer Salze, die der allgemeinen Formel (I)

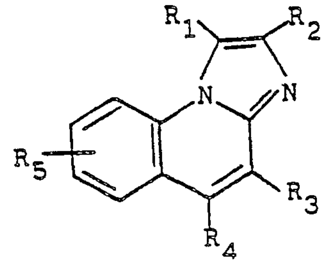

(I)

entsprechen, worin $R_1$ ein Wasserstoffatom oder ein Chlor- oder Bromatom bedeutet, $R_2$ einen Benzoylrest, einen Rest

$$-CH-O-COR_6$$

wobei $R_6$ einen Alkylrest mit 1 bis 5 Kohlenstoffatomen darstellt, einen Rest

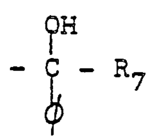

wobei $R_7$ einen Alkylrest mit 1 bis 5 Kohlenstoffatomen darstellt, oder einen Rest

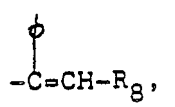

bedeutet, wobei $R_8$ ein Wasserstoffatom oder einen Alkylrest mit 1 bis 4 Kohlenstoffatomen darstellt, das Symbol $\phi$ einen Phenylrest bedeutet, $R_3$ ein Wasserstoffatom oder einen Alkylrest mit 1 bis 5 Kohlenstoffatomen bedeutet, $R_4$ einen Alkoxyrest mit 1 bis 8 Kohlenstoffatomen, eine Hydroxyrest, einen Acyloxyrest mit 2 bis 8 Kohlenstoffatomen, einen Alkylrest mit 1 bis 8 Kohlenstoffatomen bedeutet, $R_5$ einen Alkylrest mit 1 bis 8 Kohlenstoffatomen bedeutet, mit der Maßgabe, daß $R_1$ und $R_3$ nicht die Bedeutung eines Wasserstoffatoms haben können, wenn $R_2$ einen Benzoylrest darstellt und $R_4$ einen Alkoxyrest mit 1 bis 8 Kohlenstoffatomen bedeutet, dadurch gekennzeichnet, daß zur Herstellung der Produkte der Formel (I), worin $R_1$ ein Wasserstoffatom bedeutet, $R_2$ für einen Benzoylrest steht, $R_4$ einen Rest $R'_4$ darstellt, welcher einen Alkoxyrest mit 1 bis 8 Kohlenstoffatomen oder einen Alkylrest mit 1 bis 8 Kohlenstoffatomen bedeutet, $R_3$ und $R_5$ die vorstehende Bedeutung besitzen, sowie ihrer Salze man ein Produkt der Formel (II)

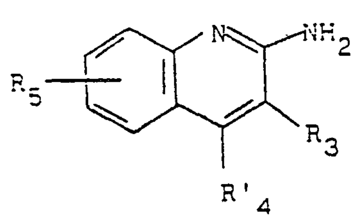

(II)

worin $R_3$, $R'_4$ und $R_5$ die angegebene Bedeutung haben, mit einem Produkt der Formel (III)

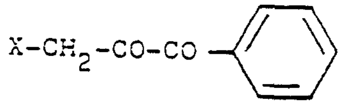

(III)

worin X ein Halogenatom darstellt, zur Reaktion bringt, um ein Produkt der Formel (IV)

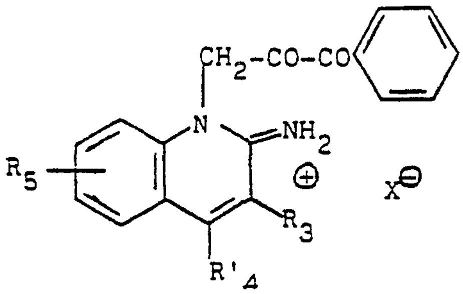

(IV)

zu erhalten, worin $R_3$, $R'_4$ und $R_5$ die angegebenen Bedeutungen besitzen, dann das erhaltene Produkt der Formel (IV) cyclisiert, um ein Produkt der Formel ($I_A$)

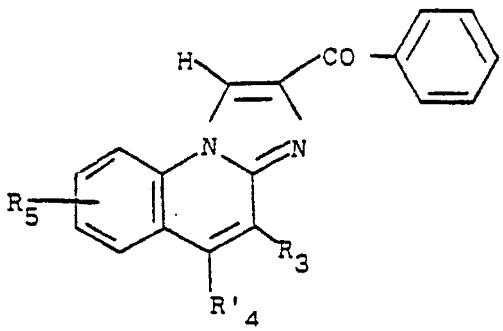

($I_A$)

zu bilden, worin $R_3$, $R'_4$ und $R_5$ die vorgenannte Bedeutung haben, das man gegebenenfalls versalzt ; zur Herstellung der Produkte der Formel (I), worin $R_1$ für ein Chlor- oder Bromatom steht, $R_2$ einen Benzoylrest bedeutet, sowie ihrer Salze man ein entsprechendes Produkt, in welchem $R_1$ ein Wasserstoffatom bedeutet, halogeniert, um ein Produkt der Formel ($I_B$)

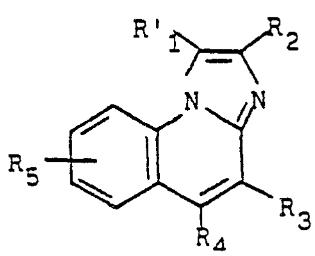

($I_B$)

zu erhalten, worin $R'_1$ ein Chlor- oder Bromatom bedeutet, $R_2$, $R_3$, $R_4$ und $R_5$ die vorgenannte Bedeutung haben, das man isoliert und gewünschtenfalls versalzt ; zur Herstellung der Produkte der Formel (I), worin $R_4$ einen Rest $R'_4$ bedeutet, $R_1$, $R_3$, $R'_4$ und $R_5$ die vorstehende Bedeutung haben und $R_2$ für einen Benzoylrest steht, sowie ihrer Salze man ein Produkt der Formel (V)

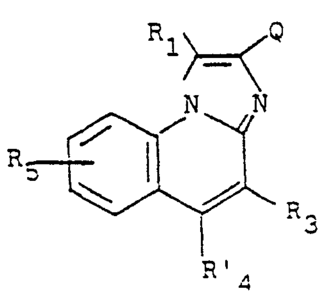

(V)

worin $R_1$, $R_3$, $R'_4$ und $R_5$ die angegebene Bedeutung haben und Q für eine Alkoxycarbonylgruppe mit 2 bis 4 Kohlenstoffatomen oder eine Aralkoxycarbonylgruppe mit 8 bis 12 Kohlenstoffatomen steht, reduziert, um ein Produkt der Formel (VI)

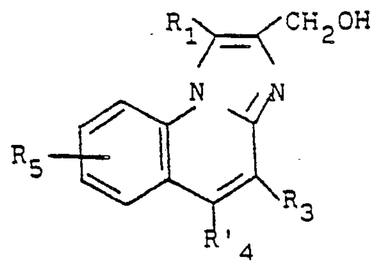

(VI)

zu erhalten, worin $R_1$, $R_3$, $R'_4$ und $R_5$ die angegebene Bedeutung besitzen, das man oxidiert, um ein Produkt der Formel (VII)

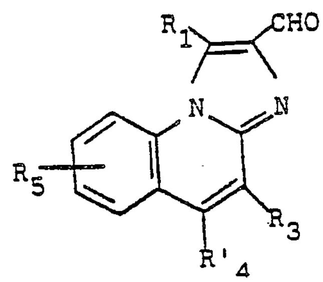

(VII)

zu erhalten, worin $R_1$, $R_3$, $R'_4$ und $R_5$ die obige Bedeutung besitzen, das man der Einwirkung eines Grignard-Reagens, gebildet aus einem Produkt der Formel (VIII)

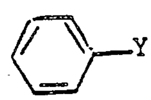

(VIII)

worin Y für ein Chlor-, Jod- oder Bromatom steht, oder der Einwirkung von Phenyllithium unterwirft, um ein Produkt der Formel (IX)

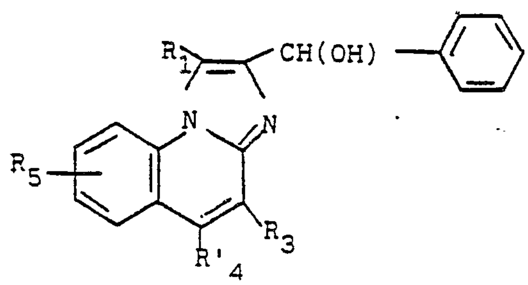

(IX)

zu erhalten, worin $R_1$, $R_3$, $R'_4$ und $R_5$ die angegebene Bedeutung haben, das man oxidiert, um ein Produkt der Formel ($I_C$)

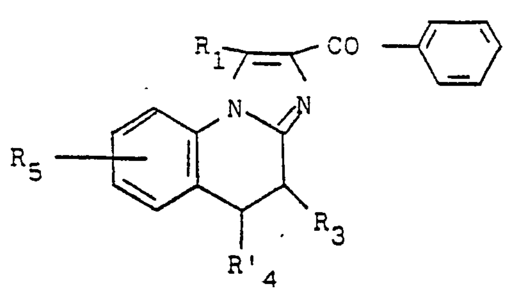

($I_C$)

zu erhalten, worin $R_1$, $R_3$, $R'_4$ und $R_5$ die vorgenannte Bedeutung haben, das man isoliert und gewünschtenfalls versalzt ; zur Herstellung der Produkte der Formel (I), worin $R_2$ für einen Rest

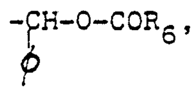

steht, $R_1$, $R_3$, $R_5$ wie vorstehend definiert sind und $R_4$ von Hydroxy verschieden ist, sowie ihrer Salze man ein Produkt der Formel

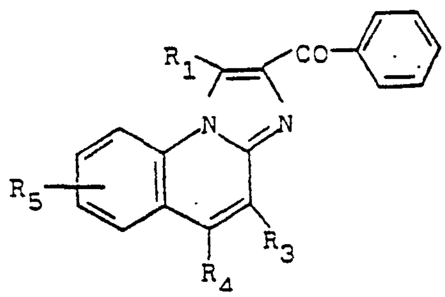

(A)

reduziert, worin $R_1$, $R_3$, $R_4$ und $R_5$ wie vorstehend definiert sind, um ein Produkt der Formel (X)

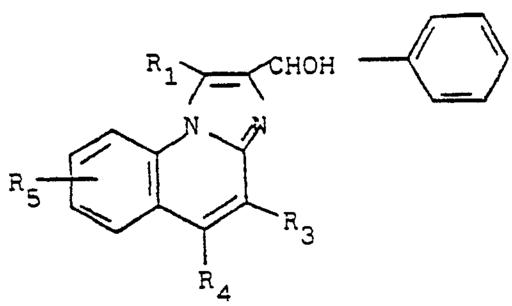

(X)

zu erhalten, worin $R_1$, $R_3$, $R_4$ und $R_5$ wie vorstehend definiert sind, das man einer Acylierungsreaktion unterwirft, um ein Produkt der Formel ($I_D$)

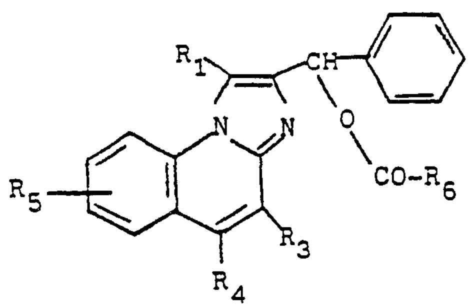

($I_D$)

zu erhalten, worin $R_1$, $R_3$, $R_4$ und $R_5$ die angegebenen Bedeutungen haben, das man isoliert und gewünschtenfalls versalzt ; zur Herstellung der Produkte der Formel (I), worin $R_2$ für einen Rest

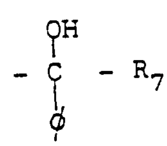

oder einen Rest

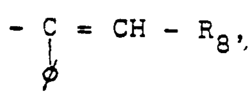

steht, $R_1$, $R_3$, $R_5$, $R_7$ und $R_8$ wie vorstehend definiert sind und $R_4$ von Acyloxy verschieden ist, sowie ihrer Salze, man ein Produkt der Formel (A), wie vorstehend definiert, mit einem Grignard-Reagens, welches aus einem Produkt der Formel (XI)

$$Y—R_7 \qquad (XI)$$

gebildet wurde, oder einem Reagens der Formel $R_7$—Li, worin Y und $R_7$ wie vorstehend definiert sind, zur Reaktion bringt, um ein Produkt der Formel ($I_E$)

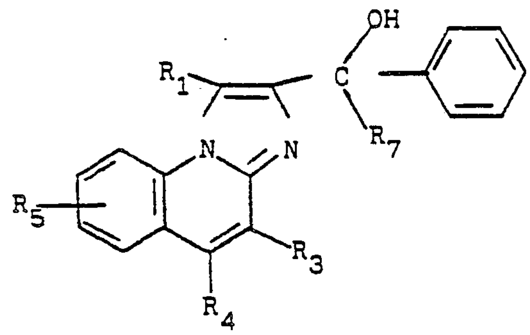

($I_E$)

zu erhalten, worin $R_1$, $R_3$, $R_4$, $R_5$ und $R_7$ die angegebenen Bedeutungen besitzen, und daß man entweder das so erhaltene Produkt der Formel ($I_E$) isoliert und gewünschtenfalls versalzt, oder das letztere dehydratisiert, in dem Fall, daß $R_7$ einen Rest $—CH_2R_8$ bedeutet, wobei $R_8$ wie vorstehend definiert ist, um ein Produkt der Formel ($I_F$)

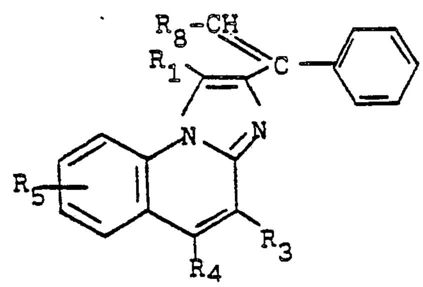

($I_F$)

zu erhalten, worin $R_1$, $R_3$, $R_4$, $R_5$ und $R_8$ die obigen Bedeutungen besitzen, welches man isoliert und gewünschtenfalls versalzt ;
zur Herstellung der Produkte der Formel (I), worin $R_1$, $R_3$ und $R_5$ wie vorstehend definiert sind, $R_2$ einen Benzoylrest oder eine Gruppe

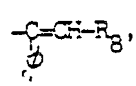

bedeutet, wobei $R_8$ wie vorstehend definiert, und $R_4$ für einen Hydroxy- oder Acyloxyrest mit 2 bis 8 Kohlenstoffatomen steht, sowie ihrer Salze, man ein entsprechendes Produkt, worin $R_4$ einen Alkoxyrest mit 1 bis 8 Kohlenstoffatomen bedeutet, hydrolysiert, um ein Produkt der Formel ($I_G$)

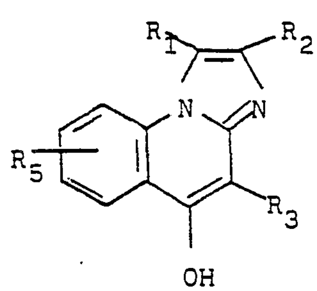

($I_G$)

worin $R_1$, $R_2$, $R_3$ und $R_5$ wie vorstehend definiert sind, zu erhalten, und daß man
entweder das so erhaltene Produkt der Formel ($I_G$) isoliert und gewünschtenfalls versalzt,
oder das letztere mit einem Halogenid der Formel (XII)

$$Hal—R_9 \qquad (XII)$$

worin $R_9$ einen Acylrest mit 2 bis 8 Kohlenstoffatomen bedeutet und Hal für ein Chlor- oder Bromatom steht, oder mit einem Säureanhydrid der Formel (XIII)

$$(R_9)_2O \qquad (XIII)$$

worin $R_9$ wie vorstehend definiert ist, zur Reaktion bringt, um ein Produkt der Formel ($I_H$)

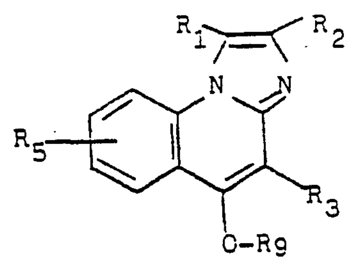

($I_H$)

zu erhalten, worin $R_1$, $R_2$, $R_3$, $R_9$ und $R_5$ die vorstehende Bedeutung haben, das man isoliert und gewünschtenfalls versalzt.

2. Verfahren gemäß Anspruch 1 zur Herstellung der Produkte der Formel (I), worin $R_1$ für ein Wasserstoffatom steht, $R_2$ einen Benzoylrest bedeutet, $R_4$ einen Rest $R'_4$ darstellt, welcher einen Alkoxyrest mit 1 bis 8 Kohlenstoffatomen oder einen Alkylrest mit 1 bis 8 Kohlenstoffatomen bedeutet, und $R_3$ und $R_5$ die vorstehende Bedeutung haben, sowie ihrer Salze, dadurch gekennzeichnet, daß man ein Produkt der Formel (II)

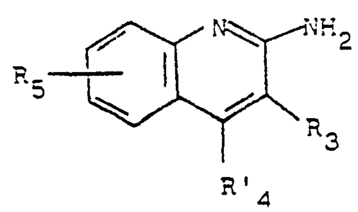

(II)

worin $R_3$, $R'_4$ und $R_5$ die angegebene Bedeutung haben, mit einem Produkt der Formel (III)

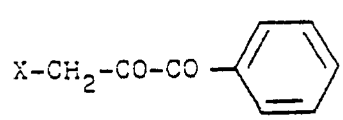

(III)

worin X ein Halogenatom darstellt, zur Reaktion bringt, um ein Produkt der Formel (IV)

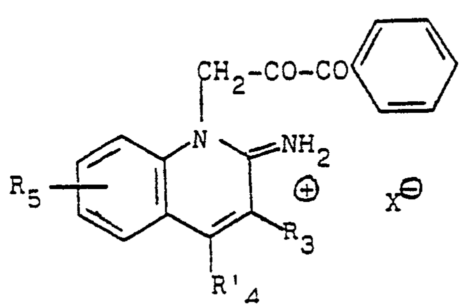

(IV)

zu erhalten, worin $R_3$, $R'_4$ und $R_5$ die angegebenen Bedeutungen besitzen, dann das erhaltene Produkt der Formel (IV) cyclisiert, um ein Produkt der Formel ($I_A$)

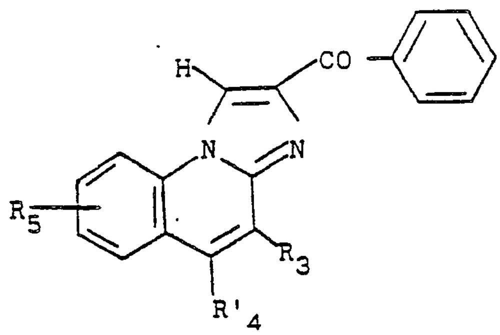

($I_A$)

zu bilden, worin $R_3$, $R'_4$ und $R_5$ die angegebene Bedeutung haben, das man gegebenenfalls versalzt.

3. Verfahren gemäß Anspruch 2, dadurch gekennzeichnet, daß

a) die Reaktion des Produkts der Formel (II) mit dem Produkt der Formel (III) in einem organischen Lösungsmittel, wie Dimethoxymethan, bewirkt wird ;

b) die Cyclisierung des Produkts der Formel (IV) durch Erhitzen in einem organischen Lösungsmittel, wie Ethanol bewirkt wird ;

c) die Reaktion des Produkts der Formel (II) mit dem Produkt der Formel (III) bis zum Produkt der Formel ($I_A$) durchgeführt wird, ohne das Produkt der Formel (IV) zu isolieren.

4. Verfahren gemäß Anspruch 1 zur Herstellung der Produkte der Formel (I), worin $R_1$ ein Chlor- oder Bromatom bedeutet und $R_2$ für einen Benzoylrest steht, sowie ihrer Salze, dadurch gekennzeichnet, daß man ein entsprechendes Produkt, in welchem $R_1$ für ein Wasserstoffatom steht, halogeniert, um ein Produkt der Formel ($I_B$)

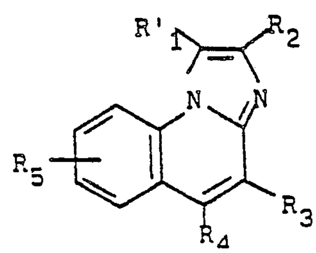

($I_B$)

zu erhalten, worin $R'_1$ für ein Chlor- oder Bromatom steht und $R_2$, $R_3$, $R_4$ und $R_5$ wie vorstehend definiert sind, das man isoliert und gewünschtenfalls versalzt.

5. Verfahren gemäß Anspruch 4, dadurch gekennzeichnet, daß

a) die Halogenierung mit Hilfe von N-Bromsuccinimid bewirkt wird, wenn man ein Produkt der Formel (I), worin $R_1$ für ein Bromatom steht, herstellen will,

b) die Halogenierung mit Hilfe von N-Chlorsuccinimid bewirkt wird, wenn man ein Produkt der Formel (I) herstellen will, worin $R_1$ für ein Chloratom steht.

6. Verfahren gemäß Anspruch 1 zur Herstellung der Produkte der Formel (I), worin $R_4$ einen Rest $R'_4$ bedeutet, $R_1$, $R_3$, $R'_4$ und $R_5$ die vorstehende Bedeutung haben und $R_2$ für einen Benzoylrest steht, sowie ihrer Salze, dadurch gekennzeichnet, daß man ein Produkt der Formel (V)

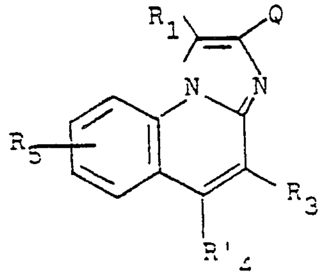

(V)

worin $R_1$, $R_3$, $R'_4$ und $R_5$ die angegebene Bedeutung haben und Q für eine Alkoxycarbonylgruppe mit 2

bis 4 Kohlenstoffatomen oder eine Aralkoxycarbonylgruppe mit 8 bis 12 Kohlenstoffatomen steht, reduziert, um ein Produkt der Formel (VI)

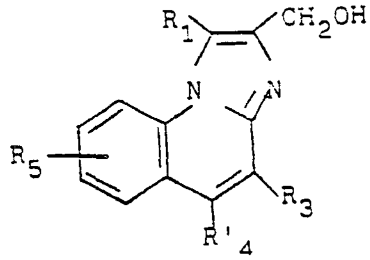

(VI)

zu erhalten, worin $R_1$, $R_3$, $R'_4$ und $R_5$ die angegebene Bedeutung besitzen, das man oxidiert, um ein Produkt der Formel (VII)

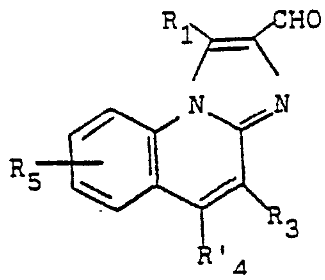

(VII)

zu erhalten, worin $R_1$, $R_3$, $R'_4$ und $R_5$ die obige Bedeutung besitzen, das man der Einwirkung eines Grignard-Reagens, gebildet aus einem Produkt der Formel (VIII)

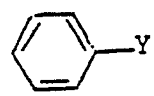

(VIII)

worin Y für ein Chlor-, Jod- oder Bromatom steht, oder der Einwirkung von Phenyllithium unterwirft, um ein Produkt der Formel (IX)

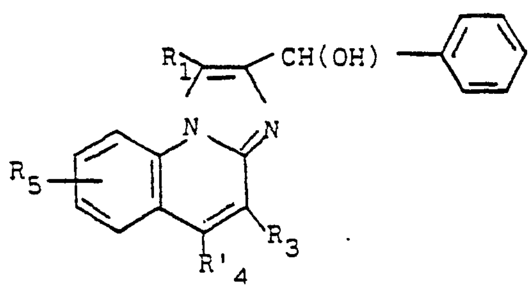

(IX)

zu erhalten, worin $R_1$, $R_3$, $R'_4$ und $R_5$ die angegebene Bedeutung haben, das man oxidiert, um ein Produkt der Formel ($I_C$)

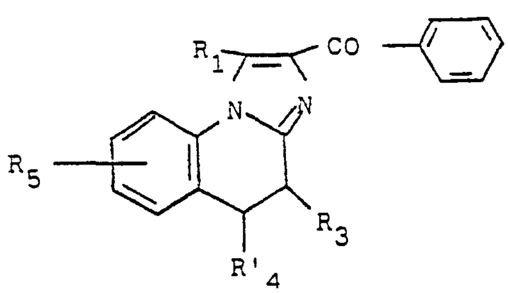

($I_C$)

zu erhalten, worin $R_1$, $R_3$, $R'_4$ und $R_5$ die vorstehende Bedeutung haben, das man isoliert und gewünschtenfalls versalzt.

7. Verfahren gemäß Anspruch 6, dadurch gekennzeichnet, daß

a) die Reduktion des Produkts der Formel (V) durch Lithiumaluminiumhydrid, der Mischung aus Natriumborhydrid und Aluminiumchlorid oder Lithiumborhydrid bewirkt wird ;

b) die Oxidation des Produkts der Formel (VI) mit Hilfe von Mangandioxid bewirkt wird oder auch mit Salpetersäure, Eisen (III)-chlorid, der Mischung Chromoxid-Pyridin oder auch durch das Oppenauer-Verfahren oder durch Dehydrierung über einem Katalysator auf Kupferbasis ;

c) die Reaktion des Produkts der Formel (VII) mit dem Grignard-Reagens oder mit Phenyllithium in Tetrahydrofuran bewirkt wird ;

d) die Oxidation des Produkts der Formel (IX) durch eine der oben angegebenen Methoden bewirkt wird.

8. Verfahren gemäß Anspruch 1 zur Herstellung der Produkte der Formel (I), worin $R_2$ einen Rest

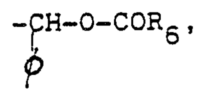

bedeutet, $R_1$, $R_3$ und $R_5$ wie vorstehend definiert sind und $R_4$ von Hydroxy verschieden ist, sowie ihrer Salze, dadurch gekennzeichnet, daß man ein Produkt der Formel

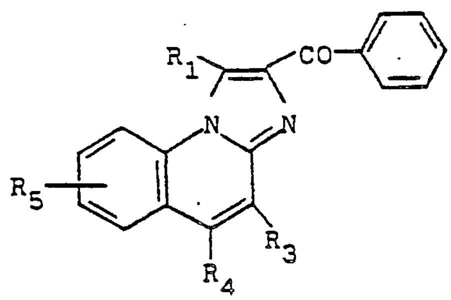

(A)

reduziert, worin $R_1$, $R_3$, $R_4$ und $R_5$ wie vorstehend definiert sind, um ein Produkt der Formel (X)

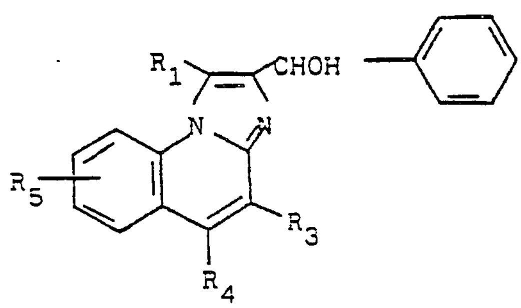

(X)

zu erhalten, worin $R_1$, $R_3$, $R_4$ und $R_5$ wie vorstehend definiert sind, das man einer Acylierungsreaktion unterwirft, um ein Produkt der Formel ($I_D$)

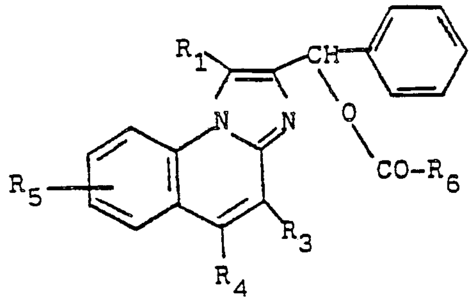

($I_D$)

zu erhalten, worin $R_1$, $R_3$, $R_4$ und $R_5$ die angegebenen Bedeutungen haben, das man isoliert und gewünschtenfalls versalzt.

9. Verfahren gemäß Anspruch 8, dadurch gekennzeichnet, daß

a) die Reduktion des Produkts der Formel (A) mit Hilfe von Natriumborhydrid in einem organischen

Lösungsmittel, wie Ethanol, durchgeführt wird ;

b) die Acylierung des Produkts der Formel (X) mit Hilfe eines Acylhalogenids, insbesondere eines Chlorids oder Bromids, in Pyridin bewirkt wird.

10. Verfahren gemäß Anspruch 1 zur Herstellung der Produkte der Formel (I) worin $R_2$ für einen Rest

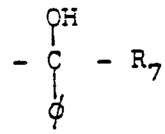

oder einen Rest

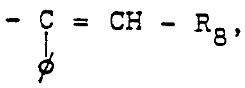

steht, $R_1$, $R_3$, $R_5$, $R_7$ und $R_8$ wie vorstehend definiert sind und $R_4$ von Acyloxy verschieden ist, sowie ihrer Salze, dadurch gekennzeichnet, daß man ein Produkt der Formel (A), wie vorstehend definiert, mit einem Grignard-Reagens, welches aus einem Produkt der Formel (XI)

$$Y\text{—}R_7 \qquad \text{(XI)}$$

gebildet wurde, oder einem Reagens der Formel $R_7$—Li, worin Y und $R_7$ wie vorstehend definiert sind, zur Reaktion bringt, um ein Produkt der Formel ($I_E$)

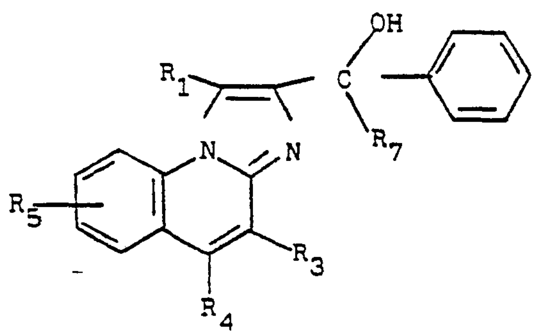

($I_E$)

zu erhalten, worin $R_1$, $R_3$, $R_4$, $R_5$ und $R_7$ die angegebenen Bedeutungen besitzen, und daß man entweder das so erhaltene Produkt der Formel ($I_E$) isoliert und gewünschtenfalls versalzt,

oder das letztere dehydratisiert, in dem Fall, daß $R_7$ einen Rest —$CH_2R_8$ bedeutet, wobei $R_8$ wie vorstehend definiert ist, um ein Produkt der Formel ($I_F$)

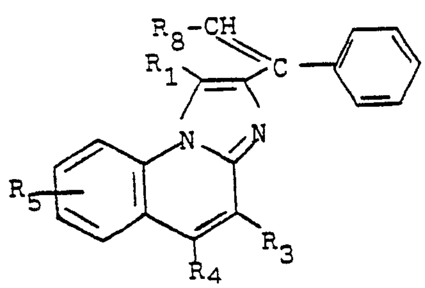

($I_F$)

zu erhalten, worin $R_1$, $R_3$, $R_4$, $R_5$ und $R_8$ die obigen Bedeutungen besitzen, welches man isoliert und gewünschtenfalls versalzt.

11. Verfahren gemäß Anspruch 10, dadurch gekennzeichnet, daß

a) die Reaktion des Produkts der Formel (A) mit dem Produkt der Formel (XI) oder dem Reagens der Formel $R_7$—Li in wasserfreiem Ether bewirkt wird ;

b) die Dehydratisierung des Produkts der Formel ($I_D$) mit Hilfe von konzentrierter Chlorwasserstoffsäure bewirkt wird.

12. Verfahren gemäß Anspruch 1 zur Herstellung der Produkte der Formel (I), worin $R_1$, $R_3$ und $R_5$ die vorstehend angegebene Bedeutung haben, $R_2$ einen Benzoylrest oder eine Gruppe —C=CH—$R_8$ bedeutet, wobei $R_8$ wie vorstehend definiert ist, und $R_4$ einen Hydroxyrest oder einen Acyloxyrest mit 2 bis 8 Kohlenstoffatomen bedeutet, sowie von deren Salzen, dadurch gekennzeichnet, daß man ein entsprechendes Produkt, in dem $R_4$ einen Alkoxyrest mit 1 bis 8 Kohlenstoffatomen darstellt, hydrolysiert, um ein Produkt der Formel ($I_G$)

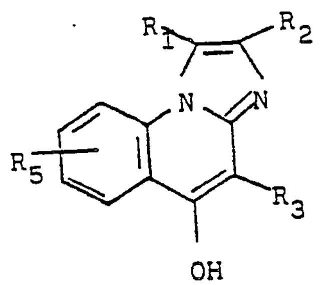

($I_G$)

worin $R_1$, $R_2$, $R_3$ und $R_5$ wie vorstehend definiert sind, zu erhalten, und daß man

entweder das so erhaltene Produkt der Formel ($I_G$) isoliert und gewünschtenfalls versalzt,

oder das letztere mit einem Halogenid der Formel (XII)

$$Hal—R_9 \qquad (XII)$$

worin $R_9$ einen Acylrest mit 2 bis 8 Kohlenstoffatomen bedeutet und Hal für ein Chlor- oder Bromatom steht, oder mit einem Säureanhydrid der Formel (XIII)

$$(R_9)_2O \qquad (XIII)$$

worin $R_9$ wie vorstehend definiert ist, zur Reaktion bringt, um ein Produkt der Formel ($I_H$)

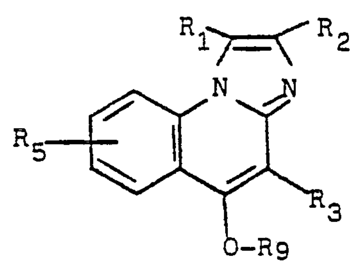

($I_H$)

worin $R_1$, $R_2$, $R_3$, $R_9$ und $R_5$ wie vorstehend definiert sind zu erhalten, das man isoliert und gewünschtenfalls versalzt.

13. Verfahren gemäß Anspruch 12, dadurch gekennzeichnet, daß

a) die Hydrolyse des Produkts, in welchem $R_4$ für einen Alkoxyrest steht, mit Hilfe von Bromwasserstoffsäure durch Erhitzen des Reaktionsmilieus zum Rückfluß während 30 h in Gegenwart von Wasser bewirkt wird ;

b) die Reaktion des Produkts der Formel ($I_G$) mit dem Halogenid der Formel (XII) oder dem Säureanhydrid der Formel (XIII) in Triethylamin bewirkt wird.

14. Verfahren gemäß einem der Ansprüche 1 bis 13, dadurch gekennzeichnet, daß man ein Ausgangsprodukt verwendet, worin $R_3$ ein Wasserstoffatom oder einen Methyl- oder Ethylrest bedeutet.

15. Verfahren gemäß einem der Ansprüche 1 bis 14, dadurch gekennzeichnet, daß man ein Ausgangsprodukt verwendet, worin $R_5$ einen Ethylrest bedeutet und $R_3$ für einen Methyl- oder Ethylrest steht.

16. Verfahren gemäß Anspruch 1, dadurch gekennzeichnet, daß man eines der Produkte der Formel I mit den folgenden Bezeichnungen herstellt :

(7-Ethyl-5-methoxy-4-methyl [imidazo [1,2-a] chinolin-2-yl]) phenylmethanon und seine Salze,

(4,7-Diethyl-5-methoxy [imidazo [1,2-a] chinolin-2-yl]) phenylmethanon und seine Salze.